# EUROPEAN PATENT APPLICATION

(11) **EP 4 617 200 A1**
(43) Date of publication of application: **17.09.2025**
(21) Application number: 24163719.8
(22) Date of filing: 15.03.2024
(51) Int. Cl.: B65D 83/00, A61M 35/00, B01F 27/91, B05C 17/01, G01F 13/00

(54) **DISPENSING APPARATUS, SYSTEM FOR MIXING AND DISPENSING MEDICAMENT AND METHOD FOR DISPENSING A SUBSTANCE**

(71) Applicant: Tickerworks, Inc., Folsom, CA 95630 (US)
(72) Inventor: PEREZ, Ramiro M., Folsom, 95630 (US)
(74) Representative: Latham, Stuart Alexander

(57) **Abstract**

Provided herein are apparatuses, systems, and methods for a metered dose dispenser with components capable of in situ mixing. In some embodiments, the subcomponents can be mixed, then subsequently assembled to generate a metered dose dispenser capable of dispensing medicaments and other cream based compositions.

## Description

### TECHNICAL FIELD

The various embodiments described herein relate to a metered dose dispenser and attachments thereof designed for use in compounding and mixing equipment to prepare certain pharmaceutical products. Moreover, the metered dose dispenser and attachments thereof can further include adapters for attaching to various mixers, and attachments including impellers.

The various embodiments described herein relate to a metered dose dispenser that can be used to dispense a medicament as an applicator or to dispense a medicament into a separate applicator.

### BACKGROUND

A typical process in creating flowable pharmaceutical compositions involves mixing suitable amounts of active ingredient, carrier, and other inert compounds in order to create a suitable flowable composition. While the intermediate steps involved in creating a flowable composition may vary, typically a mixing step is required in order to create a homogenous admixture, ensuring each measured dose delivers the correct amount of active ingredient per volume applied. Normally, composition ingredients are mixed in vessels designed for mixing, with intermediate or final compositions transferred from a mixing vessel into a dispenser to allow for administration. Transfers of intermediate or final compositions from said vessels can be a highly manual process, and due to regular residual material in the mixing vessel, or due to user or machine error, the transfer of flowable compositions from a vessel suitable for mixing to a vessel for dispensing may generate significant waste, or otherwise introduce the possibility of contamination. Thus, described herein is a metered delivery system with subcomponents capable of directly interacting with a mixer, or with attachments and accessories capable of interacting with a mixer or interacting with mixer attachments, like an impeller. Such a design offers the advantage of providing a mixing vessel that can be converted to a dispenser without the need for subsequent transfer of the flowable composition, eliminating wastage from a transfer step, the possibility of user error introduced by the transfer step, and reducing potential contamination, either from improperly sterilized transfer vessels, or contamination that may occur during the transfer step.

Moreover, such a delivery system would offer visual, tactile, and metered dispensation, which is highly desirable to either transfer the flowable composition directly into the desired body area, or as a secondary applicator designed for body cavities.

Thus, the present system and device offers a suitable mixing vessel that obviates the requirement to transfer a flowable composition, which also allows for the delivery of calibrated volumetric amounts of cream-base medicaments. The present device features a graduation area that allows consumers to visually measure a specified dose, the delivery of smaller, yet consistent volumes if desired, concurrent bi-audible, and bi-tactile features to provide dosing reassurance, and superior flexibility in the dosing of cream-base medicaments.

### SUMMARY

In various embodiments as described herein, example embodiments include at least the following aspects.

In some aspects, a dispensing apparatus includes: a housing including: an upper end; a lower end; a bottom wall positioned cross-sectionally between the upper end and the lower end the bottom wall having an aperture formed therein and a plurality of flaps surrounding the aperture and extending inward toward a center of the housing; and a plurality of primary ticks extending downward from the bottom wall of the housing; a plurality of secondary ticks extending downward from the bottom wall of the housing; a plunger positioned within the housing; a traveler in contact with the plunger, wherein the traveler extends through the aperture to move the plunger within the housing, and wherein the flaps are configured to engage the traveler; and a rotatable platform coupled to the lower end of the housing, the rotatable platform including an outer wall; an inner wall including: a plurality of inner side ticks disposed on an interior surface of the inner wall, the plurality of inner side ticks configured to interact with one or more of the plurality of primary ticks; and a plurality of outer side ticks disposed on an exterior surface of the inner wall, the plurality of inner side ticks configured to interact with the secondary ticks.

In some aspects, the rotatable platform further includes a traveler silo in contact with at least a portion of the traveler, such that rotation of the rotatable platform relative to the housing causes the traveler to rotate.

In some aspects, the traveler is configured to move upward in response to rotation of the rotatable platform through the aperture.

In some aspects, the traveler includes a head in contact with an inner surface of the traveler silo.

In some aspects, the traveler silo is configured to move upward in response to rotation of the rotatable platform through engagement between the head of the traveler and the inner surface of the traveler silo.

In some aspects, the inner wall of the rotatable platform is taller than the outer wall.

In some aspects, the traveler is longer than the length of the traveler silo.

In some aspects, a portion of the traveler silo extends into the aperture in the bottom wall of the housing.

In some aspects, the plurality of flaps includes four flaps positioned 90 degrees apart around the aperture.

In some aspects, a dispensing apparatus further includes a cap, the cap including a label surface of sufficient size for a prescription label to be affixed to the label surface.

In some aspects, a system for mixing and dispensing a medicament includes: a housing including: an upper chamber; a lower chamber; and a bottom wall dividing the upper chamber and the lower chamber, the bottom wall having an aperture formed therein and a plurality of flaps surrounding the aperture and extending inward toward a center of the housing; a plurality of primary ticks extending downward from the bottom wall of the housing; and a plurality of secondary ticks extending downward from the bottom wall of the housing, wherein a wall of the housing extends below the plurality of primary ticks and the plurality of secondary ticks, wherein the housing is configured to be inserted into a mixing apparatus, a bottom of the housing contacting a bottom of the mixing apparatus.

In some aspects, the system further includes: a driver complex including a rotatable platform and a traveler, wherein the driver complex is configured to be coupled to the mixing complex, wherein when the driver complex is coupled to the mixing complex, the plurality of flaps in the bottom wall are configured to engage the traveler, wherein rotating the rotatable platform causes the traveler to rotate upward through the aperture and move a plunger within the housing upward in the upper chamber of the housing and dispense the medicament through the at least one dispensing outlet.

In some aspects, the mixing complex further includes a volume indicator below the plunger in the upper chamber, the volume indicator including an indicator lip.

In some aspects, the system further includes a mixing lid to close the housing.

In some aspects, the mixing lid includes an impeller aperture and an impeller seal.

In some aspects, the mixing lid includes a plurality of vertical lift taps and a lift notch on each side of the lift tab.

In some aspects, the system further includes a dispensing lid coupled to the upper chamber, the dispensing lid including a dispensing outlet.

In some aspects, the mixing apparatus is a centrifugal mixer.

In some aspects, the techniques described herein relate to a method for dispensing a substance including: providing a substance within a housing of a dispensing apparatus; rotating a rotatable platform coupled to a lower end of the housing; moving in an upward direction, via the rotation of the rotatable platform, a traveler extending through an aperture of a bottom wall of the housing; engaging a plunger within the housing with the traveler; moving the plunger in an upward direction; and dispensing a substance through an aperture of a dispensing lid coupled to the upper end of the housing.

In some aspects, the techniques described herein relate to a method, the method further including: engaging a plurality of primary ticks extending from the bottom wall of the housing with a plurality of inner side ticks disposed on an interior surface of an inner wall of the rotatable platform; and creating a clicking sound caused by the engagement of the plurality of primary ticks with the plurality of inner side ticks as the rotatable platform rotates.

In one aspect described herein, a dispensing apparatus comprises a housing comprising: an upper end; a lower end; a bottom wall positioned cross-sectionally between the upper end and the lower end the bottom wall having an aperture formed therein and a plurality of flaps surrounding the aperture and extending inward toward a center of the housing; and a plurality of primary ticks extending downward from the bottom wall of the housing; a plurality of secondary ticks extending downward from the bottom wall of the housing; a plunger positioned within the housing; a traveler in contact with the plunger, wherein the traveler extends through the aperture to move the plunger within the housing, and wherein the flaps are configured to engage the traveler; and a rotatable platform coupled to the lower end of the housing, the rotatable platform comprising an outer wall; an inner wall comprising: a plurality of inner side ticks disposed on an interior surface of the inner wall, the plurality of inner side ticks configured to interact with one or more of the plurality of primary ticks; and a plurality of outer side ticks disposed on an exterior surface of the inner wall, the plurality of inner side ticks configured to interact with the secondary ticks.

In some embodiments, the rotatable platform further comprises a traveler silo in contact with at least a portion of the traveler, such that rotation of the rotatable platform relative to the housing causes the traveler to rotate.

In some embodiments, the traveler is configured to move upward in response to rotation of the rotatable platform through the aperture.

In some embodiments, the traveler comprises a head in contact with an inner surface of the traveler silo.

In some embodiments, the traveler silo is configured to move upward in response to rotation of the rotatable platform through engagement between the head of the traveler and the inner surface of the traveler silo.

In some embodiments, the inner wall of the rotatable platform is taller than the outer wall.

In some embodiments, the traveler is longer than the length of the traveler silo.

In some embodiments, a portion of the traveler silo extends into the aperture in the bottom wall of the housing.

In some embodiments, the plurality of flaps comprises four flaps positioned 90 degrees apart around the aperture.

In some embodiments, the dispenser further comprises a cap, the cap including a label surface of sufficient size for a prescription label to be affixed to the label surface.

In another aspect described herien, the techniques or devices described herein relate to a dispensing apparatus including: a housing including: an upper end; a lower end; a bottom wall positioned cross-sectionally between the upper end and the lower end, thereby forming an upper chamber and a lower chamber within the housing, the bottom wall having an aperture therethrough and a plurality of flaps extending inward toward a longitudinal axis through the center of the housing; and a plurality of primary ticks and secondary ticks extending downward from the bottom wall of the housing; a plunger positioned within the upper chamber of the housing; a traveler including a head, a threaded cylinder, and a tip, wherein the traveler is configured to extend through the bottom wall of the housing to move the plunger within the upper chamber of the housing, wherein the flaps in the bottom wall are configured to engage the threaded cylinder of the traveler as the traveler travels upwards through the bottom wall; a rotatable platform configured to be coupled to the lower end of the housing, the rotatable platform including concentric walls including: an outer wall that forms the exterior surface of the rotatable platform; a middle wall including a plurality of inner side ticks on an interior surface of the middle wall configured to interact with the primary ticker tabs extending from the bottom wall of the housing, and further including a plurality of outer side ticks on an exterior surface of the middle wall configured to interact with the secondary ticker tabs extending from the bottom wall of the housing; and an inner wall, wherein the inner wall forms a traveler silo configured to house at least a portion of the traveler.

In some aspects, the techniques or devices described herein relate to a dispensing apparatus, wherein axially rotating the rotatable platform relative to the housing is configured to axially rotate the traveler relative to the housing.

In some aspects, the techniques or devices described herein relate to a dispensing apparatus, wherein axially rotating the traveler moves the traveler upward within the traveler silo and rotates the traveler through the flaps in the bottom wall of the housing.

In some aspects, the techniques or devices described herein relate to a dispensing apparatus, wherein the traveler head includes a larger diameter than the diameter of the threaded cylinder and the tip, and wherein the traveler head includes a smaller diameter than the diameter of the traveler silo.

In some aspects, the techniques or devices described herein relate to a dispensing apparatus, wherein the traveler silo is sized to engage and rotate the traveler head and to permit translational upward movement of the traveler head within the traveler silo upon axially rotation of the rotatable platform.

In some aspects, the techniques or devices described herein relate to a dispensing apparatus, wherein the inner wall of the rotatable platform is taller than the middle wall and the middle wall is taller than the outer wall.

In some aspects, the techniques or devices described herein relate to a dispensing apparatus, wherein the inner wall is a hexagon shape, and wherein the head of the traveler is a hexagon shape.

In some aspects, the techniques or devices described herein relate to a dispensing apparatus, wherein the traveler is longer than the length of the traveler silo in the inner wall.

In some aspects, the techniques or devices described herein relate to a dispensing apparatus, wherein the inner wall of the rotatable platform includes a top rim, wherein when the apparatus is assembled the top rim is configured to extend into a plane of the aperture in the bottom wall of the housing.

In some aspects, the techniques or devices described herein relate to a dispensing apparatus, wherein the plurality of flaps includes four flaps, the flaps positioned 90 degrees apart around the aperture in the bottom wall.

In some aspects, the techniques or described herein relate to a dispensing apparatus comprising a cap, the cap configured to be secured to the housing of the dispensing apparatus, the cap including a label surface, wherein the label surface is of sufficient size for a prescription label to be affixed to the label surface.

In some aspects, the techniques or devices described herein relate to a system for mixing and dispensing a medicament including: a mixing complex configured to be received in a mixing apparatus for mixing the medicament, the mixing complex including a housing and a plunger, wherein the housing includes: an upper chamber; a lower chamber; and a bottom wall dividing the upper chamber and the lower chamber, wherein the bottom wall includes an aperture therethrough centered around a longitudinal axis of the housing, wherein the bottom wall includes a plurality of flaps extending toward the longitudinal axis, wherein the plunger includes an upper sealing lip and is positioned in the upper chamber of the housing and disposed above the bottom wall, wherein the sealing lip is configured to contact an interior surface of the housing, wherein the mixing complex is configured to receive one or more components of the medicament in the upper chamber above the plunger; a driver complex including a rotatable platform and a traveler, wherein the driver complex is configured to be coupled to the mixing complex, wherein the traveler includes a head and a threaded cylinder, wherein the rotatable platform includes a traveler silo and the traveler is configured to be at least partially disposed in the traveler silo, a dispensing lid including at least one dispensing outlet, wherein when the driver complex is coupled to the mixing complex, the flaps in the bottom wall are configured to engage the threaded cylinder of the traveler, wherein rotating the rotatable platform causes the traveler to rotate upward through the flaps and move the plunger upward in the upper chamber of the housing and dispense the medicament through the at least one dispensing outlet.

In some aspects, the techniques or devices described herein relate to a system, wherein the traveler silo is hexagon shaped and the head of the traveler is hexagon shaped.

In some aspects, the techniques or devices described herein relate to a system, wherein the traveler silo is polygon shaped and the head of the traveler is polygon shaped.

In some aspects, the techniques or devices described herein relate to a system, wherein the rotatable platform includes concentric walls including: an outer wall that forms the exterior surface of the rotatable platform; and an inner wall that forms the traveler silo.

In some aspects, the techniques or devices described herein relate to a system, wherein the rotatable platform includes a middle wall as part of the concentric walls, the middle wall disposed between the outer wall and the inner wall.

In some aspects, the techniques or devices described herein relate to a system, wherein the inner wall is taller than the middle wall, and the middle wall is taller than the outer wall.

In some aspects, the techniques or devices described herein relate to a system, wherein the mixing complex further includes a volume indicator below the plunger in the upper chamber, the volume indicator including an indicator lip.

In some aspects, the techniques or devices described herein relate to a system, further including a mixing lid to close the housing complex when medicament is mixed in the housing complex.

In some aspects, the techniques or devices described herein relate to a system, wherein the mixing lid includes an impeller aperture and an impeller seal.

In some aspects, the techniques or devices described herein relate to a system, wherein the mixing lid includes a plurality of vertical lift taps and a lift notch on each side of the lift tab.

In some aspects, the techniques or devices described herein relate to a system, wherein the dispensing lid includes only one dispensing outlet and the dispensing outlet is configured to engage a separate medicinal applicator.

In some aspects, the techniques or devices described herein relate to a system, wherein around the exterior of the outer wall, upper line markings are arranged every 90 degrees, and wherein, around the exterior of the lower end of the housing, lower line markings are arranged every 18 degrees.

### BRIEF DESCRIPTION OF THE DRAWINGS

The various embodiments are illustrated by way of example, and not by way of limitation, in the figures of the accompanying drawings in which:
FIG. 1 depicts structural elements of a metered dose dispenser 100, presented in a separable view to show the potential orientation of certain components relative to one another.
Figs. 2A and 2B depict cross sectional views of housing complex 108, either alone (FIG. 2A), or along with components that may be placed inside the housing complex (FIG. 2B).
FIG. 3 depicts a view of housing complex 108 as shown when viewing the housing complex from the lower end.
FIGs. 4A and 4B, show housing complex 108 configured to accept the traveler 112 when assembling the device.
FIGs 5A, 5B, and 5C depict partial profile views of a lid attachment mechanism.
FIG. 5D depicts a side profile view of a lid coupled to the housing complex.
FIG. 6A depicts a cross section view of the plunger 110 and volume indicator 114.
FIG. 6B depicts an angled, non-cross section view of plunger 110 and volume indicator 114.
FIG. 6C depicts volume indicator 114 placed within the dimensions of plunger 110.
FIG. 6D depicts volume indicator 114 placed within the dimensions of housing complex 108.
FIGs. 7A-7C depict cross section views of an embodiment of the drug indicator.
FIGs. 7D-7E depict embodiments of the drug indicator.
FIG. 8 depicts an embodiment of the rotatable platform.
FIGs. 9A, 9B, 9C, and 9D each depict various angled views of rotatable platform 140.
FIGs. 10A, 10B, and 10D each depict various angled views of rotatable platform 140.
FIG. 10C depicts a cross sectional, side view of the rotatable platform 140.
FIG. 11A and 11B depict an angled view of rotatable platform 140 along with traveler 112.
FIG. 12 and FIG 13 each depict cross sectional views of an embodiment of an apparatus comprising the rotatable platform coupled to the housing complex 108.
FIG. 14A and FIG. 14B each depict cross sectional views of the drug indicator 136 placed below the bottom wall 210, and within the boundaries of housing wall 206.
FIG. 15A depicts a profile view of the mixing complex 160 comprising the housing complex 108 and the plunger 110.
FIG. 15B depicts a profile view of driver complex 170 comprising the rotatable platform 140 and traveler 112.
FIG. 15C depicts a profile view of mixing complex 160 with accessories.
FIG. 16A depicts a profile view of a combined apparatus comprising mixing complex 160 attached to driver complex 170 comprising the rotatable platform 140 and traveler 112.
FIGs. 16B-C depicts profile views of a combined apparatus attached to a barrel for later dispensing of medicament.
FIGs. 17A, 17B, 17C, 17D and 18A, 18B, and 18C depict a mixing lid 1702 with varying angular and cross-sectional views.
FIG. 19 depicts mixing lid 1702 configured to accept an impeller shaft 1904
FIG. 20 depicts an apparatus for mixing comprising housing complex 108, mixing lid 1702, and the impeller 1900.
FIG. 21 depicts a cross section of a mixing assembly for use with EMP mixers.
FIGs. 22A-C depicts various views of an adapter 2202.
FIG. 23 depicts an assembly comprising adapter 2202, housing complex 108, and flat lid 2302.
FIG. 24 depicts a cross section view of the assembly comprising adapter 2202, housing complex 108, and flat lid 2302.
FIG. 25 depicts an isometric view of a metered dose dispenser 100.
FIG. 26 depicts an isometric view of a metered dose dispenser 100 with a cap 2602.
FIG. 27 depicts a top isometric view of a cap 2602.
FIG. 28 depicts a bottom isometric view of a cap 2602.
FIG. 29 depicts a front view of a metered dose dispenser 100.
FIG. 30 shows an isometric view of a rotatable platform 140.

### DETAILED DESCRIPTION

In the following description, for purposes of explanation, numerous specific details are set forth in order to provide a thorough understanding of the various embodiments. It will be evident, however, to one of ordinary skill in the art that the various embodiments may be practiced without these specific details. Moreover, all parts of the example embodiments described herein can be made of rigid plastic, metal, or any combination of suitable medical grade materials. In some embodiments, the material is polypropylene.

The various embodiments described herein relate to a metered dose dispenser and attachments thereof designed for use in compounding and mixing equipment to prepare certain pharmaceutical products. Moreover, the metered dose dispenser and attachments thereof can further include adapters for attaching to various mixers, and attachments including impellers.

Some embodiments described herein relate to a dispenser for flowable medicaments, specifically, a unidirectional rotatable platform containing a traveler that together comprise a driver complex, which is compatible with a mixing complex. Rotation of the driver complex relative to the mixing complex rotates the traveler through flaps in a bottom wall of the mixing complex, which then drives the movement of a plunger. The mixing complex is designed to be compatible with mixer units in order to mix flowable compositions. Upon assembly of the mixing complex with the rotatable platform, the dispenser is capable of dispensing flowable medicaments upon rotation of the rotatable platform relative to the housing. By rotating the rotatable platform, the plunger rises and a predetermined amount of flowable composition discharges from one or more dispensing outlets within the lid of the dispenser.

In some embodiments, a topical, visual, bi-audible and bi-tactile to the senses metered applicator with calibrated line demarcations on both the housing complex and the rotatable platform wherein an 18 degree axial rotation of the rotatable platform with respect to the housing complex, constitutes one metered dispensation and translates to roughly a 1 /20th milliliter of volumetric output is described in detail herein.

The various embodiments described herein thus eliminate the necessity of using two separate vessels for mixing and dispensing, instead using a novel housing complex compatible with a driver complex (including the rotatable platform and traveler) to form a metered dosing dispenser apparatus. The advantages of such a device allow for the precise, metered dispensing of certain cream-based medicaments, or other medicaments in liquid form. Moreover, the advantages of the housing complex allows for in situ mixing without the need for transferring or changing vessels, thus minimizing waste and reducing the potential for user error during compounding or reducing cross contamination.

In some embodiments, a mixing apparatus is adapted to attach to components that enable the overall apparatus to dispense metered dose topical medicaments. In some embodiments, the apparatus is capable of attaching to components to aid in mixing, including adapters, and impeller attachments. The apparatus features a housing complex, wherein an internally loaded medicament compound is pushed out via a plunger operably attached to a traveler component.

Several embodiments are disclosed, in which a novel device employs a unidirectional rotational mechanism where visual, audible, and tactile elements work together synergistically for the delivery of calibrated volumetric amounts of topical cream-based medicaments of a specified density, or any other flowable material if warranted. The interaction of primary and secondary ticker tabs stemming from a bottom wall of the housing complex, and corresponding inner and outer side ticks stemming from a wall of the rotatable platform; which, allows for the delivery of specific volumetric amounts of flowable medicaments, is also provided.

Moreover, the interaction and involvement of selective ticker tabs stemming from housing complex and corresponding inner and outer side ticks stationed on the rotatable platform; which, are responsible for creating two different types of audible sounds and tactile sensations to the user depending on the angular displacement of the rotatable element against the house from a predetermined point of reference will be disclosed in detail.

The term "housing", "house", or "housing complex" will be used to denote a barrel, and one or more internal features as described herein. As can be appreciated, the housing complex can be operably coupled to a mixer, in order to mix any ingredients or intermediate formulations contained within. The housing complex, after mixing, can then be coupled to a driver complex (including a rotatable platform and traveler), in order to form a metered dose dispenser, or dispensing apparatus. The housing complex can also fit within an adapter, which is shaped to accept the housing complex, and to fit within the mixing platform of a mixer device.

Some embodiments disclosed herein offer the benefit of better mixing of the medicament in the apparatus by allowing a lower center of gravity for the medicament within the apparatus. As can be appreciated, the addition of the driver complex to the mixing complex (meaning the housing complex with at least the plunger in the upper chamber) increases the dimensions of the overall assembled apparatus, including the overall length of the assembly. Thus, the mixing complex alone without the driver complex has a shorter height than the assembled apparatus, and a point within the mixing complex will have a lower center of gravity when the driver complex is not attached to the mixing complex. Specifically, the reservoir in the mixing complex (meaning the area in the upper chamber of the housing complex above the plunger) will have a lower center of gravity before the mixing complex is assembled with driver complex to form the fully assembled apparatus. The embodiments of the disclosure herein allow the mixing complex alone (meaning without the addition of the driver complex) to be used in a mixing device such as a centrifugal mixer. It has been determined that having a lower center of gravity generates more efficient and better mixing when the mixing complex is placed within the mixing device such as a centrifugal mixer when compared to an apparatus where the reservoir is at a higher center of gravity. Additionally, mixing without the driver complex may save on space when fitting the housing complex to a mixer, allowing compounders the ability to mix larger quantities of compounded preparations without the need to utilize larger mixing equipment.

One example embodiment of the dispensing apparatus includes a unidirectional rotatable platform that engages a traveler that can be slipped into an upper chamber of the housing complex and secured in place by cooperation of a plurality of flaps and retainers located within an aperture along a bottom wall. Once inside, the traveler interacts with plunger, or with a volume indicator operably connected to a plunger, such that the plunger fits tightly against the inner wall of said housing upper chamber. Upon axial rotation of the rotatable platform against the house, visual, audible and tactile sensations can be perceived by the user at, for example, every 18 degrees of rotation. However, the type of sound depends if the rotation of the platform against the housing complex causes activation of primary or secondary ticker tabs stemming from the housing complex, which interact with inner or outer side ticks stemming from the rotatable platform. When a primary ticker tab is directed to an inner side tick (first sound) or a secondary ticker tab is further directed to an outer side tick (second sound) since different ticker tabs are involved and interact with inner and outer side ticks depending on the displacement of the rotatable platform against the house from a predetermined point of reference. In one embodiment, activation of the primary ticker tabs stemming from the housing complex interact with inner side ticks stemming from the rotatable platform to cause a first sound. In some embodiments, secondary ticker tabs stemming from the housing complex interact with outer side ticks stemming from the rotatable platform to cause a second sound. In some embodiments, every 5^{th} click (translating to a 90 degree displacement of the rotatable platform against the housing complex), both the primary and secondary ticker tabs interact with inner side ticks and outer side ticks respectively to produce a more accentuated sound; wherein, a first sound and a second sound occur almost simultaneously. The plunger rises and pushes upward the flowable contents of such chamber, exiting through a dispensing outlet located at the center of a dispenser lid, which is secured to the upper end of the housing providing a surface to apply the cream or gel directly onto the skin, or to another container. A removable cap with a plug to retard evaporation of the cream-base medicament may also be used. As the platform rotates, the user is able to view marked volumetric indicators along the side of the housing. A threaded traveler (rotated to the right generally) interacts with the plunger equipped with a top and/or bottom edge seal useful in preventing a cream smudge trail or any visible cream from being left behind and causes it to rise. A bi-audible mechanism is in place for determining set volumetric amounts dispensed upon an 18° clockwise rotation of the rotatable platform against the house. A 90°, 180°, 270°, or a 360° degree displacement of the rotatable platform against the house from a predetermined point of reference produces a distinct audible and tactile sensation on the user, referred here as the second sound. Likewise, displacement other than a 90°, 180°, 270°, or 360° degrees from a predetermined point of reference, produces a different yet distinct audible and tactile sensation on the user, referred here as the first sound. An 18° rotation of the rotatable platform translates to a different line marking on the exterior wall of the housing complex in some embodiments. It follows that a 90° rotation of the rotatable platform against the housing complex translates to moving past five line markings on the housing complex in some embodiments. In some embodiments, the device has been configured to deliver roughly one gram of a specified cream of a specific density (or 1.03 g of water at 25° C.) for every 360° rotation of the rotatable platform against the house.

### Metered Dose Dispenser

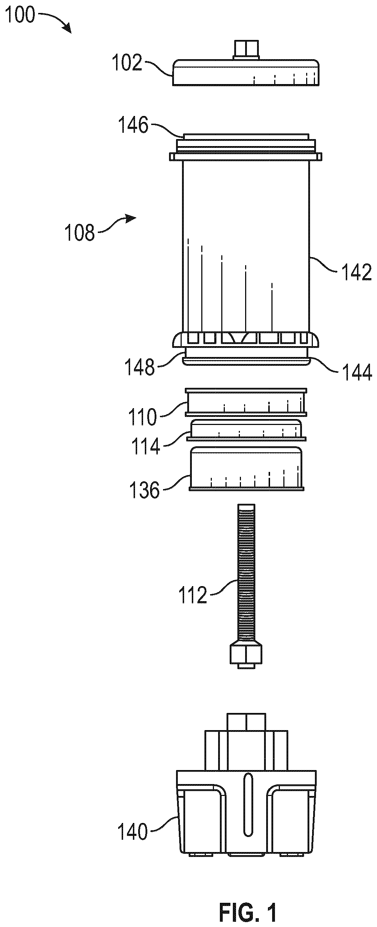

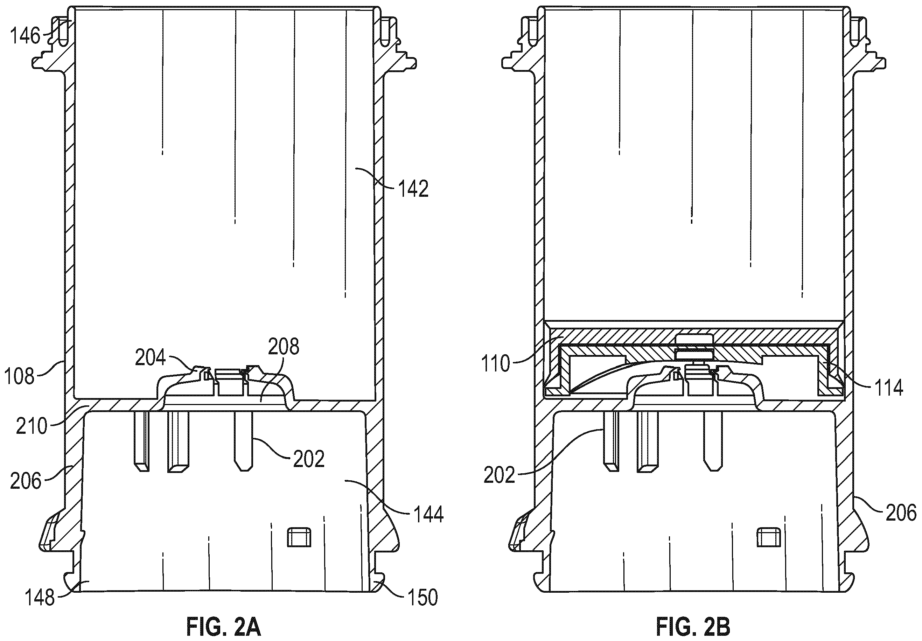

FIG. 1 depicts structural elements of a metered dose dispenser 100, including a dispensing lid 102, a housing complex 108, a plunger 110, a volume indicator 114, a drug indicator 136, a traveler 112, and a rotatable platform 140. The figure is presented in a separable view to show the potential orientation of certain components relative to one another. Although the plunger 110 and volume indicator 114 are shown below the housing complex 108 in the figure, in one embodiment, the plunger 110 and volume indicator 114 are positioned in an upper chamber 142 of the housing complex 108 (see e.g., Figs. 2A-2B). The plunger 110 may be designed to sit atop the volume indicator 114, wherein both the plunger 110 and the volume indicator 114 are configured to move up and down within the housing complex 108. In some embodiments, the dispensing lid 102 rests atop the housing complex 108. Additionally, the drug indicator 136 can be fitted below the volume indicator 114 and the plunger 110 to visually indicate the medicament identity or type. In one embodiment, the drug indicator is positioned in a lower chamber 144 of the housing complex 108 (see e.g., Figs. 2A-2B). In some embodiments, the position of the drug indicator 136 is static and does not move relative to the movement of the plunger 110 or the volume indicator 114. In some embodiments, the traveler 112 is designed to reside substantially within the rotatable platform 140 and is configured to apply an upward force on the plunger 110 within the housing complex 108. Various components of the dispenser will be described in greater detail below.

The housing complex (or simply, housing) 108 comprises an upper end 146 and a lower end 148. The upper end 146 and lower end 148 are each designed to accept and attach certain components as detailed herein, including the dispensing lid 102 and/or a rotatable platform 140. When certain components are attached, the result is the creation of a fully or substantially enclosed assembly for the topical application of a medicament. Moreover, the housing complex 108 is configured to accept other components that reside substantially within the housing complex 108, such as the volume indicator 114 and the plunger 110.

### Housing Complex

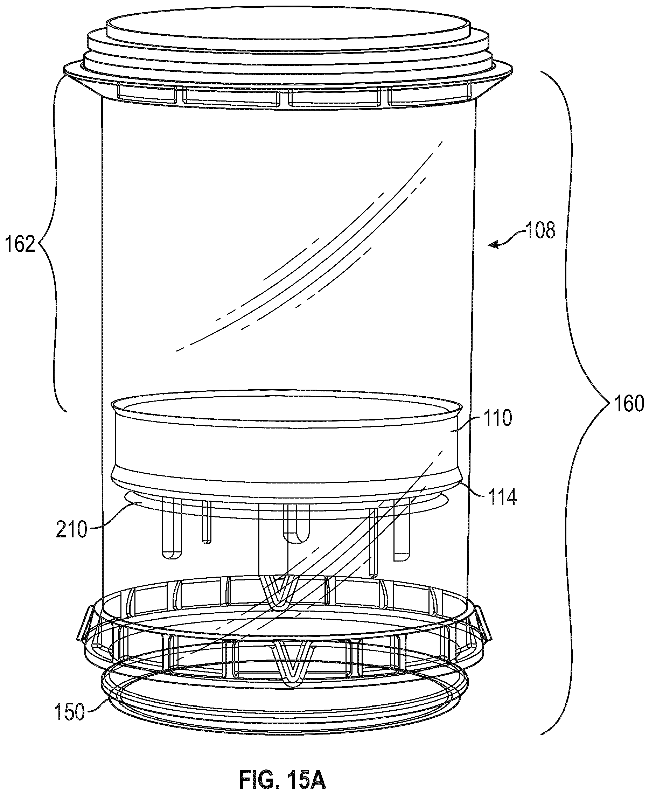

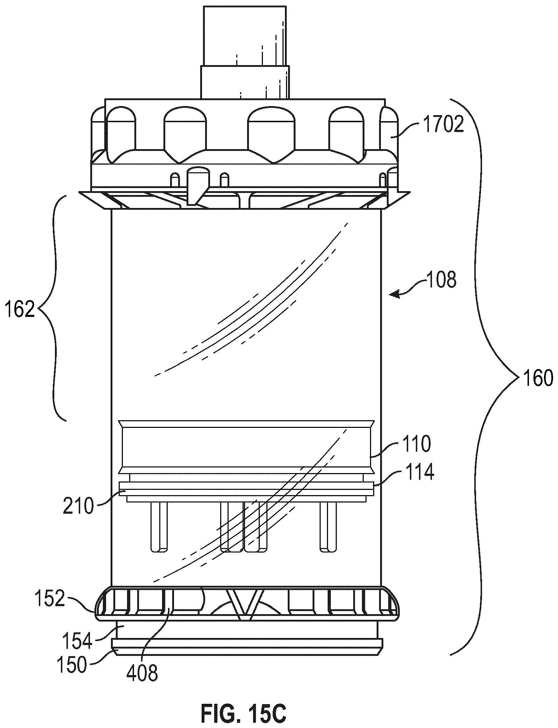

Figs. 2-5 show the housing complex 108 which serves as a container or reservoir for medicament and medicament ingredients, and which comprises certain structural features to allow for the attachment of additional components to create an enclosed or substantially enclosed assembly. Moreover, the housing complex 108 is configured to accept volume indicator 114 and the plunger 110, which in some embodiments, are present within the housing complex 108 when contents are mixed (and in such instances may be referred to as the mixing complex 160 (see discussion of Figs 15A and 15C below)). The housing complex 108 (when assembled as the mixing complex 160) is configured to receive and house contents placed within the housing complex 108 and to mix the contents using, for example, a centrifugal mixing device, an impeller based mixer, or any other method to mix, homogenize, or emulsify certain compositions.

Figs. 2A and 2B depict cross sectional views of the housing complex 108, either alone (FIG. 2A), or along with components which sit inside the housing complex (FIG. 2B).

With reference to FIG. 2A, the housing complex 108 comprises primary ticker tabs 202, flaps 204, the housing wall 206 and an aperture 208. Additionally, as shown in FIG. 2B, the volume indicator 114 and plunger 110 are configured fit inside the upper chamber 142 of the housing complex 108. As shown in FIG. 2A, the housing complex 108 can include a bottom wall 210 that serves as a cross-sectional barrier to separate the housing complex 108 into the upper chamber 142 and the lower chamber 144. Moreover, in some embodiments, bottom wall 210 comprises an aperture 208 therethrough.

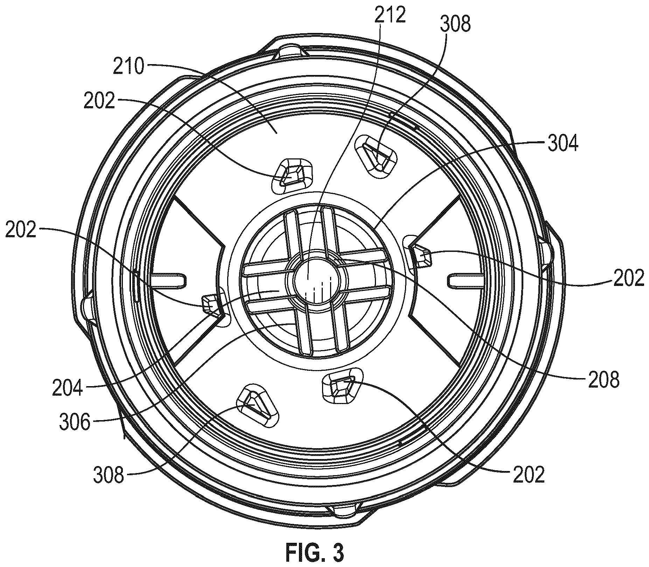

FIG. 3 depicts an end view of the housing complex 108 as shown from the lower end 148. Notably, aperture 208 is located within the bottom wall 210, and the bottom wall 210 is configured to cross-sectionally divide the housing complex 108 into an upper chamber 142 and a lower chamber 144. In some embodiments, the bottom wall 210 therefore divides the housing complex 108 into two chambers, and the housing wall 206 extends in the lower direction beyond the bottom wall 210 to form the lower chamber 144. The bottom wall 210 comprises an interior aperture wall 304, which is in some embodiments may be an annular shape. The bottom wall 210 comprises a plurality of flaps 204 and a plurality of traveler retainers 306, which can be positioned to extend inward from the aperture wall 304 or an upper surface of the bottom wall 210 towards a longitudinal axis through the center of the housing complex 108. A plurality of primary ticker tabs 202 and secondary ticker tabs 308 can extend from the lower surface of the bottom wall 210 in a downward direction. In some embodiments, the primary ticker tabs 202 and the secondary ticker tabs 308 are configured to interact with corresponding side ticks resident on the rotatable platform 140, to produce a sound and/or tactile feedback corresponding to rotational movement of the rotatable platform 140.

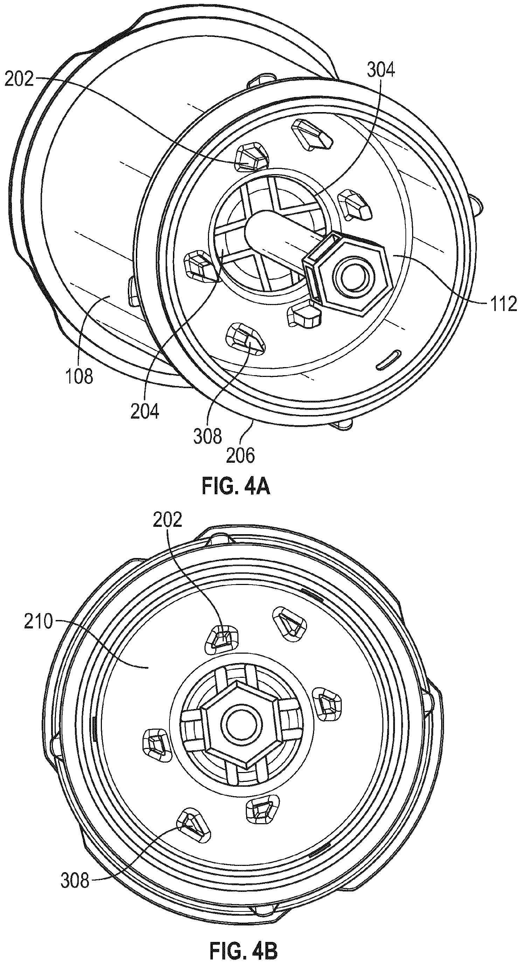

In one embodiment, as shown in FIG. 3, the housing complex 108 includes four primary ticker tabs 202 and two secondary ticker tabs 308 extending downward from the bottom wall 210. In other embodiments, however, the housing complex 108 may include a different number of primary ticker tabs 202, such as 1, 2, 3, 5, 6, 7, 8, 9, 10, etc. And in other embodiments, the housing complex 108 may include a different number of secondary ticker tabs 308, such as 1, 3, 4, 5, 6, 7, 8, 9, 10, etc. As shown in FIG. 3 (see also FIGs. 4A and 4B), the primary ticker tabs 202 are positioned closer to the aperture 208 in the middle of the bottom wall 210 (as well as the longitudinal axis through the center of the housing complex 108) than the secondary ticker tabs 308, and the primary ticker tabs 202 and may be spaced apart equally around the circumference of the aperture 208. Thus, the secondary ticker tabs 308 are at a further distance from the aperture 208 than the primary ticker tabs 202 and may be spaced apart equally around the circumference of the aperture 208. Referring to FIGs. 4A, 15A, and 15C, the primary ticker tabs 202 and secondary ticker tabs 308 do not extend downward past the lower end 148 of the housing complex 108, and thus are positioned entirely within the lower chamber 144 of the housing complex 108.

In one embodiment, and as depicted in FIG. 3, the bottom wall 210 comprises four flaps 204 and four traveler retainers 306, which may extend from the aperture wall 304. In other embodiments, the device may include a different number of flaps 204 and traveler retainer 306. The bottom wall 210 may have, for example, 2, 3, 4, 5, 6, 7, 8, 9, 10, flaps 204 and 0, 2, 3, 4, 5, 6, 7, 8, 9, 10 traveler retainers 306. If the plurality of flaps 204 includes four flaps, as shown in Fig. 3, they may be positioned at 90 degrees around the aperture 208. Likewise, if the plurality of traveler retainers 306 includes four traveler retainers, they may be positioned at 90 degrees around the aperture 208. In some embodiments, the flaps 204 may be substantially rectangular in shape and the traveler retainers 306 may be substantially triangular in shape. The traveler retainers may be alternated with the flaps 204 in the aperture 208, and the arrangement may include slits or gaps 205 between each flap 204 and the traveler retainers 306 on each side of the flap 204. In some embodiments, the flaps 204 and the traveler retainers 306 generally abut each other around the aperture 208 and do not include gaps between them. In some embodiments, the bottom wall 210 includes only flaps 204 and no traveler retainers 306. The plurality of flaps 204 (alone or in combination with the traveler retainers 306) form a traveler aperture 212 through the bottom wall 210.

In some embodiments, as shown in FIGs. 4A and 4B, the flaps 204 are configured to accept the traveler 112 through the traveler aperture 212 when assembling the device. In some embodiments, the ends of the flaps 204 may form the traveler aperture 212 as a non-contiguous ring on the inside of, and concentric with, the larger aperture 208. In this manner, the combination of the flaps 204 can serve as a ring that receives the threads on the traveler 112 as shown in FIGs. 4A and 4B and thus facilitates advancing the traveler 112 upward or downward through the bottom wall 210 of the housing complex 108 when the traveler 112 is axially rotated relative to the housing complex 108. In some embodiments, traveler 112 is capable of axial rotation in only one direction to advance the traveler upwards through the bottom wall 210 of the housing complex 108. As described below, the combination of structural elements as described herein facilitate unidirectional movement for each plane of movement - including upwards motion of the traveler 112, and clockwise motion of the traveler 112 relative to the housing complex 108. In some embodiments, the flaps 204 may have curved end surfaces that create part of the traveler aperture 212, and the curved end surfaces can aid in contacting and securing the traveler 112. The traveler aperture 212 may have a diameter that is smaller than the diameter of the shaft 1104 of the traveler 112.

In some embodiments, the flaps 204 are configured, before the traveler 112 is inserted into the flaps 204, to be substantially planar with the bottom wall 210 as they extend inward toward the longitudinal axis of the housing complex 108. In some embodiments, the flaps 204 are configured, before the traveler 112 is inserted into the flaps 204, to project upward at an angle into the upper chamber 142 from the plane of the bottom wall 210 as they extend inward toward the longitudinal axis of the housing complex 108.

In some embodiments, the flaps 204 are flexible, such that the traveler 112 may push the flaps 204 upward when it is threaded into and gripped by the flaps 204 when advancing through the bottom wall 210. Once the traveler 112 begins to be rotated through the traveler aperture 212 and the flaps 204 are pushed up, the flaps 204 then grip or interact with threads on the traveler 112. In some embodiments, the flaps 204 may only be temporarily pushed up by the rotation of the traveler 112 and may again move to a lower position if, for example, the traveler was permitted to move in the downward direction. In some embodiments, the flaps 204 are substantially rigid and do not move significantly when gripping the traveler 112 when it is advanced through the bottom wall 210 and traveler aperture 212.

In some embodiments, the flaps 204 are assisted in keeping the traveler in place by the traveler retainers 306. The traveler retainers 306 extend from the aperture wall 304 towards the center of the aperture 208 and serve to prevent off-axis movement by the traveler 112. In some embodiments, the traveler retainers 306 do not contact the traveler 112 when it is aligned properly and advancing correctly through bottom wall 210 via the flaps 204 gripping the traveler 112. In some embodiments, the traveler retainers 306 are configured to contact the traveler 112 along with the flaps 204 to assist in gripping and securing the traveler 112 as it advances through the bottom wall 210.

In another embodiment (not shown), the bottom wall 210 does not include a plurality of flaps in the bottom wall 210, but instead has an aperture therethrough that has a contiguous interior wall that is at least partially threaded to receive the traveler 112 as it is advanced through the bottom wall 210. In another embodiment (not shown), the bottom wall 210 comprises an aperture therethrough to receive the traveler 112, and includes one or more slits positioned in the bottom wall surface and running outwardly from the aperture wall, and the aperture through the bottom wall may be threaded or not threaded.

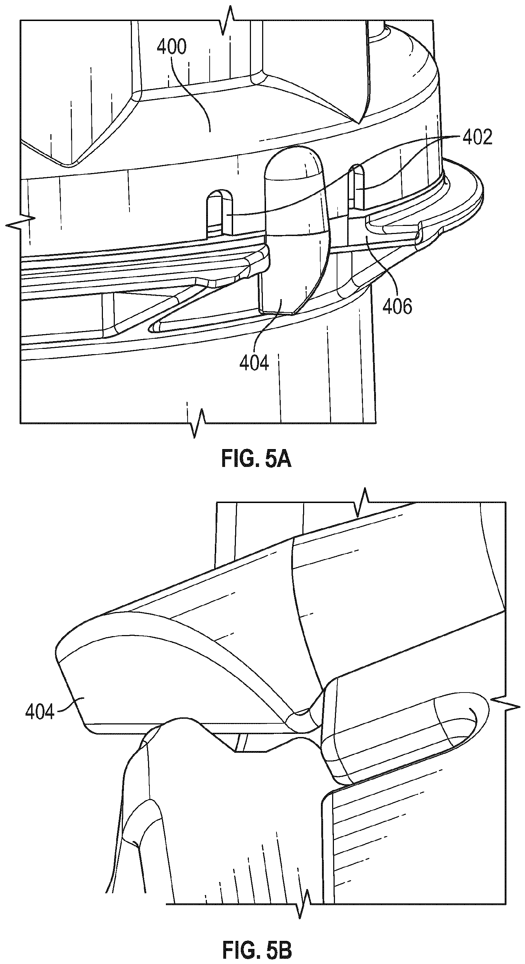

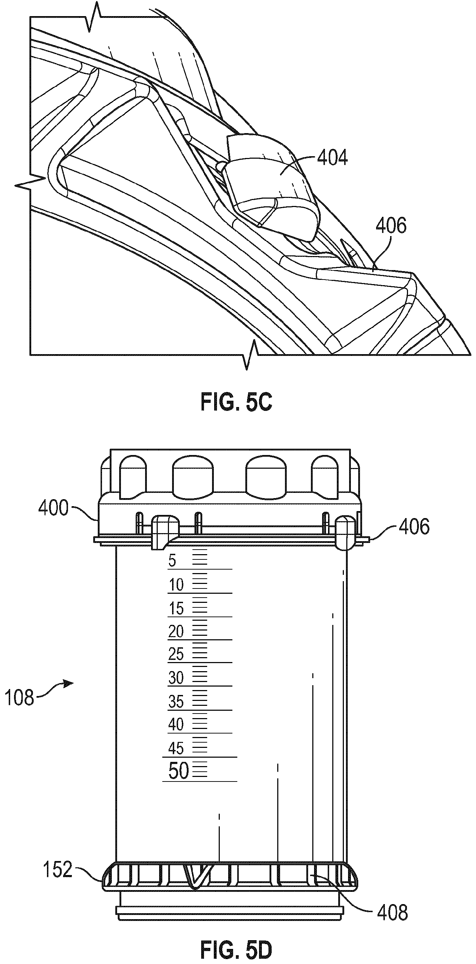

FIGs 5A, 5B, and 5C depict partial profile views of a lid attachment mechanism as exemplified in notched lid 400. FIG. 5D depicts the attachment of notched lid 400 to housing complex 108. The notched lid 400 comprises lift notches 402 and a vertical lift tab 404. The upper portion of housing complex 108 comprises an axial lifter rim 406. In some embodiments, the axial lifter rim 406 is configured to have an angular or sloping portion, such that the movement of the vertical lift tab 404 along a sloped portion of the axial lifter rim decreases the total frictional force holding the notched lid 400 to the housing complex 108. Thus, applying force and moving the vertical lift tab 404 along the axial lifter rim 406 with an angular protrusion can cause the notched lid 400 to lift off, and thus detach from the housing complex 108. The presence of lift notches 402 can further impart flexibility to the vertical lift tab 404. In some embodiments, the notched lid 400 serves to dispense flowable medicament, including highly viscous medicament, or medicament with large particulate matter embedded within. As can be appreciated from FIG. 5D, the housing complex 108 can further comprise upper line markings 408, which may be part of a raised ring 152 (discussed below with respect to FIG. 15C). The upper line markings 408 may be spaced at regular intervals around the perimeter of the housing complex 108, including, for example, every 18 degrees around the perimeter. In some embodiments, the upper line markings 408 can be used to mark relative rotation of the rotatable platform 140.

### Plunger and Volume Indicator

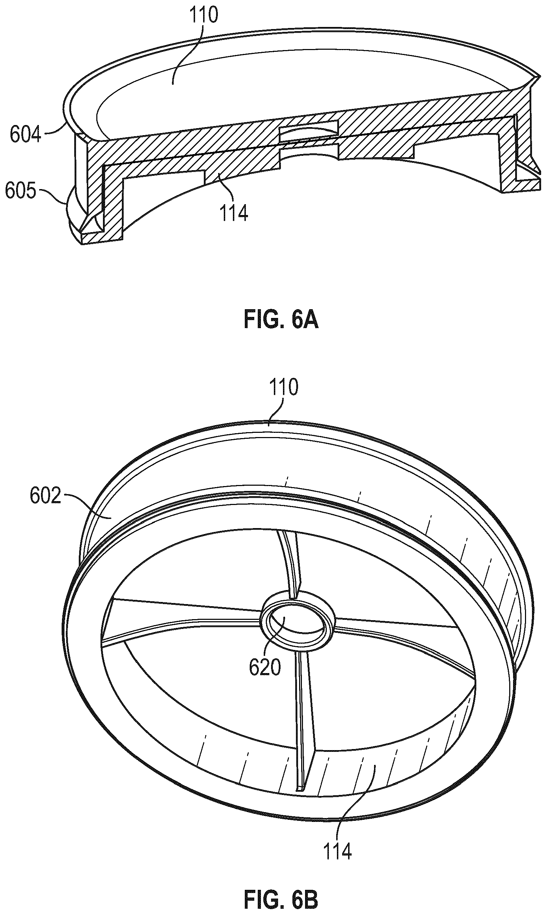

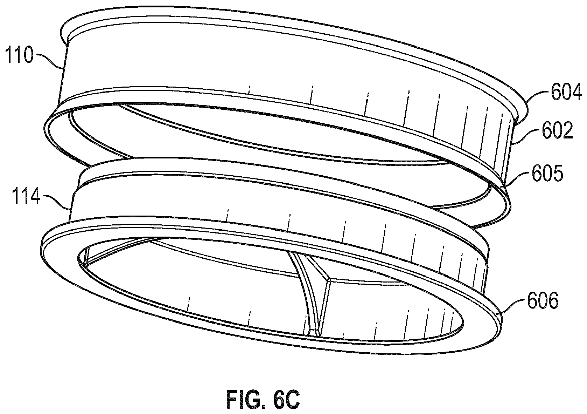

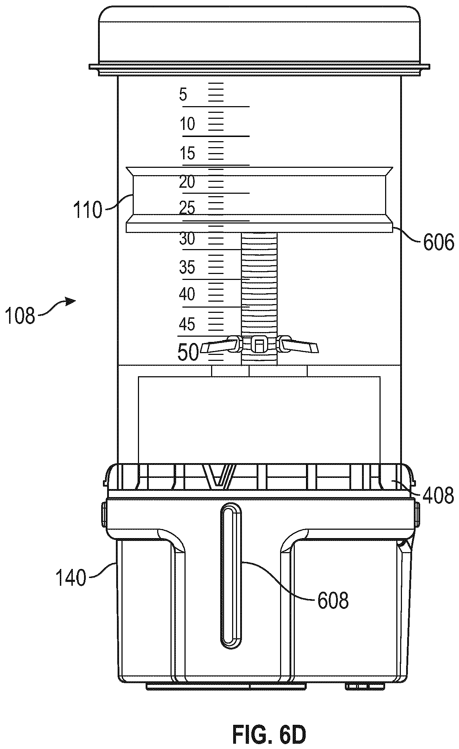

FIG. 6A is a cross section view of the plunger 110 and volume indicator 114. FIG. 6B is a perspective view of the same, showing the bottom side of the volume indicator 114. FIG. 6C depicts how volume indicator 114 fits within the dimensions of plunger 110. FIG. 6D depicts how the volume indicator 114 can be used to show the volume remaining of a compound dispensed from the housing complex 108. The volume indicator 114 moves with the plunger as the plunger is moved due to movement of the traveler in response to rotation of the rotatable platform. The indicator lip 606 of the volume indicator moves along a graduated measurement scale, and the indicator lip 606 is visible through the housing. The indicator lip 606 of the volume indicator aligns with one of the graduations on the graduated scale to indicate the volume of medicament remaining in the dispenser. In some embodiments, not shown, the volume indicator has a trough right through the middle of the indicator lip and around the circumference of the housing complex, the trough serving to better indicate a more precise volume remaining inside the chamber to the end user. In some embodiments, the plunger 110 comprises plunger side wall 602, as well as sealing lip 604 and lower lip 605.

As can be appreciated from FIGs. 6A and 6C, the plunger 110 is d above volume indicator 114, including above the flat surface of the volume indicator 114. The plunger 110 and volume indicator 114 are intended to be placed in the upper chamber 142 of the housing complex 108 above the bottom wall 210. In some embodiments, the plunger 110 is fitted over the volume indicator 114 and they are configured to move together within the housing complex 108. When placed within the housing complex 108, the plunger 110 is configured to push flowable compositions upwards and dispense medicaments from the device.

The plunger 110 is sized and configured so that when the plunger 110 is placed within the housing complex 108, flowable material cannot flow downward past the sealing lip 604, even when the plunger 110 is in motion and traveling upwards within the housing complex 108. In some embodiments, the traveler 112 is configured to push upward on the volume indicator 114, to move the volume indicator 114 and plunger 110 together in the housing complex 108. The volume indicator 114 can include a ring 620 on the underside that receives the top of the traveler 112. As the volume indicator 114 and the plunger 110 travels in the housing complex 108, the total volume available for a composition within housing complex 108 decreases. In some embodiments, the volume indicator 114 comprises an indicator lip 606, which is configured to indicate the available volume for a flowable composition within housing complex 108.

As shown in FIG. 6A, the plunger's lower lip 605 can rest on the top of the indicator lip 606 of the volume indicator 114. As shown in FIG. 6D, the indicator lip 606 can line up with markings on housing complex 108 to indicate the current available volume for a flowable composition within housing complex 108. As can be appreciated in the example from FIG. 6D, approximately 27mL of volume is available for a flowable composition based on the position of the indicator lip 606. In some embodiments, the metered dose dispenser 100 does not include a separate component as the volume indicator 114, and in such cases a portion of the plunger, such as the sealing lip 604 or lower lip 605, can serve to mark the volume of medicine remaining in the housing complex 108. Moreover, FIG. 6D further illustrates how a lower line marking 608 can be used in conjunction with upper line markings 408. Rotation of the rotatable platform 140 can be tracked by the relative motion of the lower line marking 608 relative to the markings from the upper line markings 408. In some embodiments, the lower line markings 608 may be spaced at regular intervals around the rotatable platform, including every 90 degrees.

### Drug Indicator

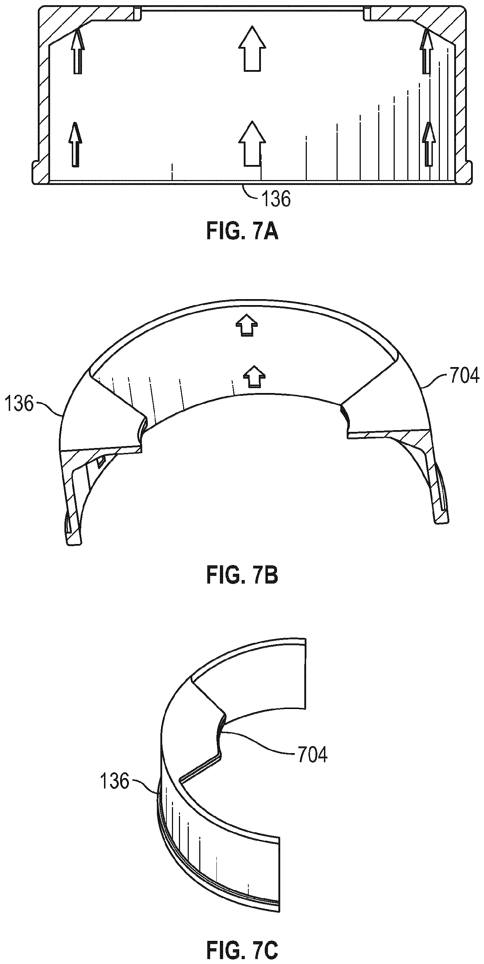

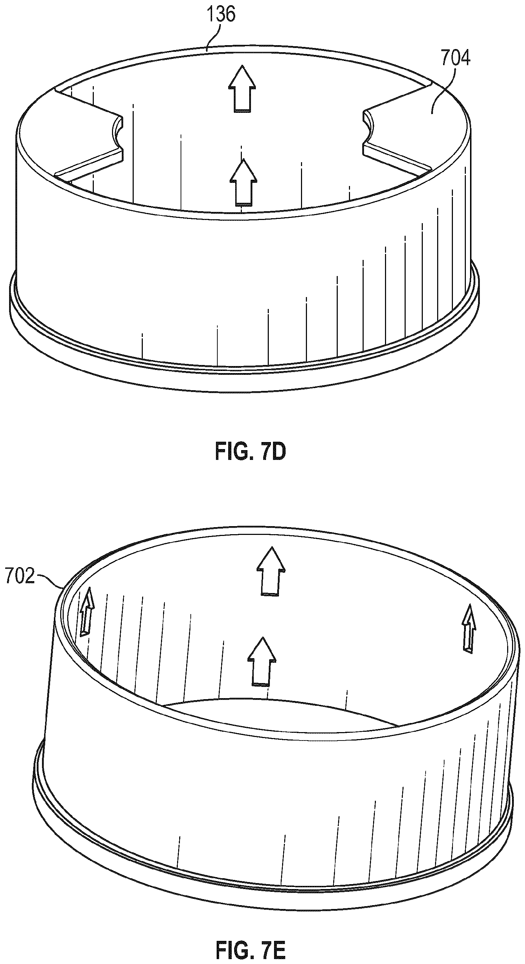

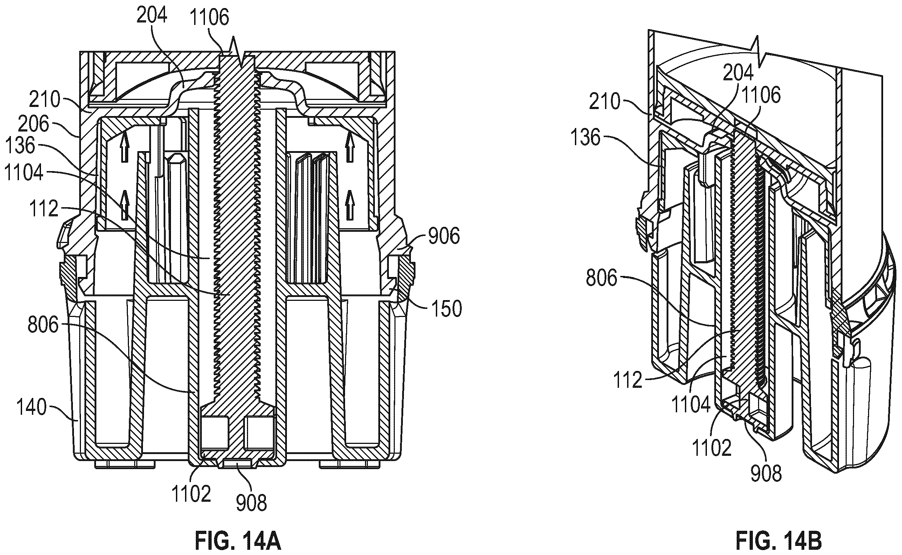

FIGs. 7A-7C depict cross-section views of the drug indicator 136. In some embodiments, the drug indicator 136 is configured to be placed below the bottom wall 210 and be visually identifiable through the clear material of housing wall 206. Moreover, drug indicator 136 comprises an aperture therein to allow the traveler 112 to pass therethrough. The drug indicator 136 can indicate visually, by text on the surface or based on the color, any variety of drug related properties, including medicament identity, class, warning labels, administration schedule, or preparation date. In some embodiments, the drug indicator 136 allows for the rapid, visual identification of a drug compound. As an example, a testosterone drug compound may have a red colored drug indicator 136 and/or include a "testosterone" label on the outward surface. In some embodiments, a label can be affixed to the drug indicator 136. For reference, FIG. 14A and FIG. 14B each depict cross sectional views of the drug indicator 136 placed below the bottom wall 210, and within the boundaries of housing wall 206. In this manner, an outer frame of the drug indicator 136 is fittingly positioned in the lower chamber 144. The upward arrows on the drug indicator 136 shown in the figures indicates the direction for the user to insert the drug indicator 136 in the lower chamber 144. FIG. 7D depicts an embodiment of the drug indicator 136. Likewise, FIG. 7E depicts an alternative embodiment of a drug indicator 702, which functions similarly to indicate visually any variety of drug related properties. As can be appreciated, one or more overhangs 704 serve to arrest axial rotational movement of the drug indicator 136, by interacting with one or more primary ticker tabs 202 and/or secondary ticker tabs 308 to fix the drug indicator 136 in place.

A compounding pharmacist can, for example, place a drug indicator as illustrated throughout FIGs 7A-7E to indicate the contents of housing complex 108. Since the drug indicator 136 is a separate component which can be inserted into housing complex 108, the metered dose dispenser 100 can be visually marked or labeled with a high degree of customizability. In some embodiments, a user (e.g., a pharmacist) can use a certain color of a drug indicator 136 for a specific type of medicament.

### Rotatable Platform

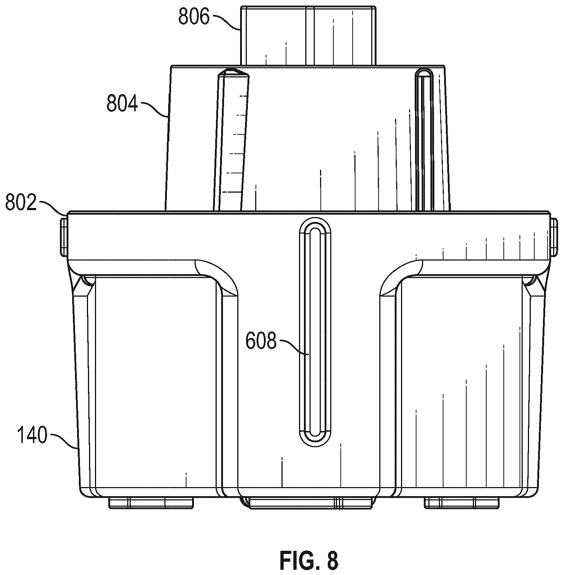

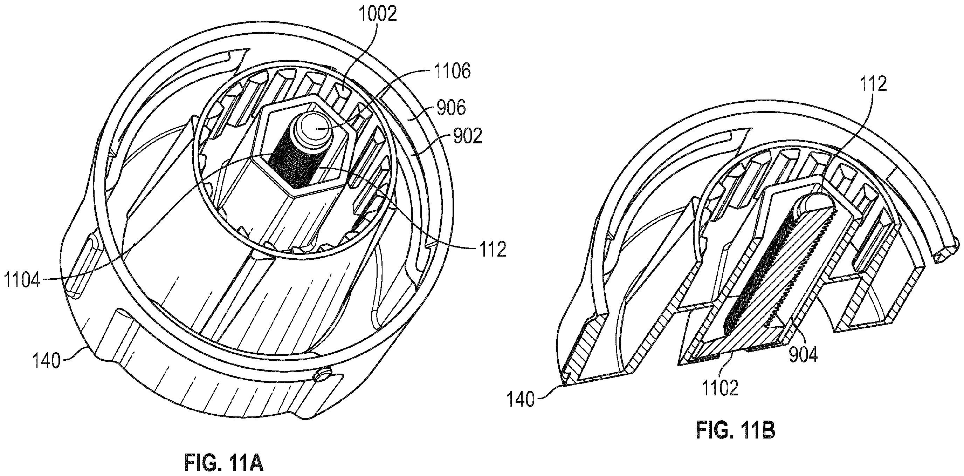

FIGs. 8-14 depict various embodiments of the rotatable platform 140. As depicted in FIG. 8, which is a profile view of rotatable platform 140, the rotatable platform can comprise concentric walls, including an outer wall 802, middle wall 804, and inner wall 806. In some embodiments, the inner wall 806 has a greater height than the middle wall 804 (i.e. extends beyond), and the middle wall 804 has a greater height (i.e. extends beyond) than the outer wall 802. In some embodiments, the inner wall 804 can be a traveler silo for housing and interacting with at least a portion of the traveler. The names outer wall, inner wall, and middle wall are exemplary only. The structure of the inner wall 806 is configured to accept and partially encompass traveler 112, as illustrated in FIG. 11A and 11B.

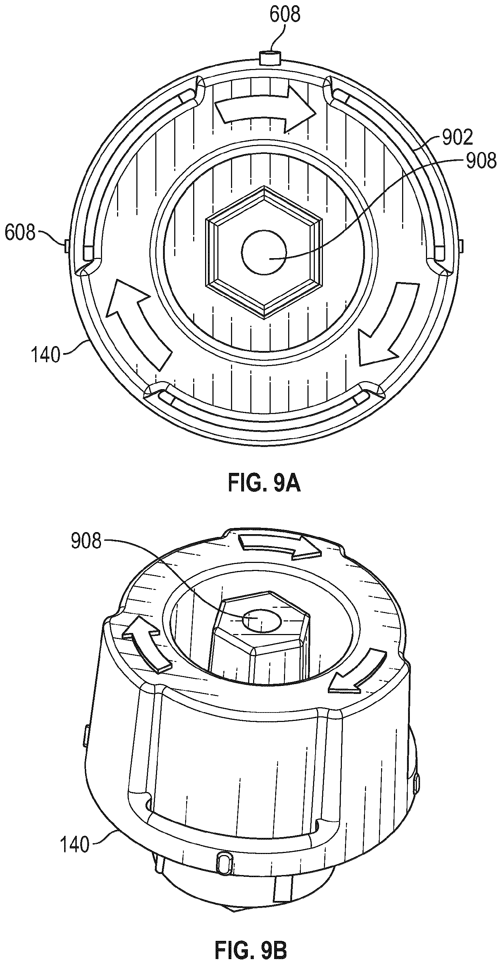

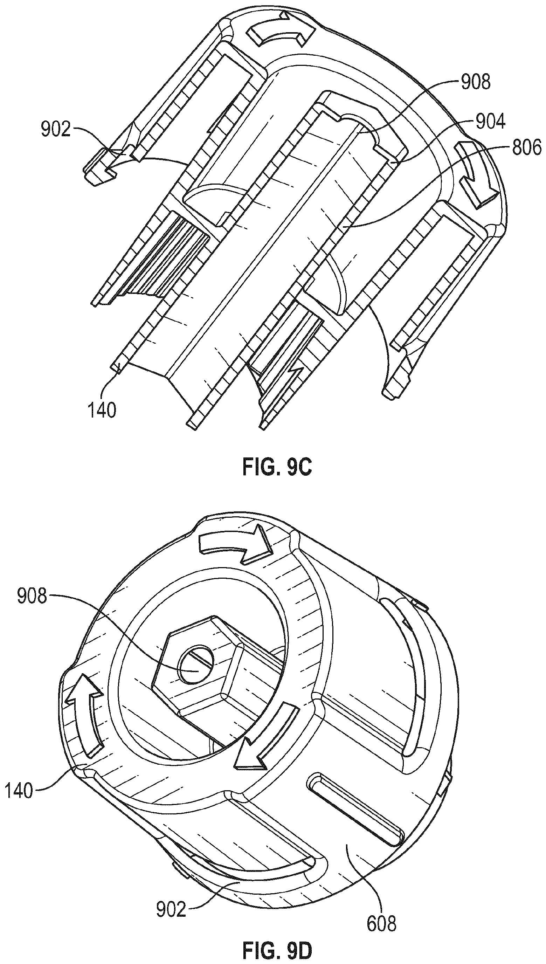

FIGs. 9A, 9B, and 9D each depict various angled views of rotatable platform 140. Moreover, FIG. 9C depicts a cross sectional, side view of the rotatable platform 140. As shown in FIGs. 9A-9D, in some embodiments the rotatable platform 140 further comprises one or more acoustic voids 902. In some embodiments, the acoustic voids 902 are resident on the outer wall 802. The acoustic voids 902 serve to amplify and modulate the sound produced by the interaction of primary ticker tabs 202 and inner side ticks 1002, as detailed herein. The rotatable platform 140 can also include a traveler access aperture 908 in the bottom of the inner wall 806 that passes through to the traveler silo 904, allowing access to the traveler 112 in the rotatable platform 140, as explained in further detail below. The traveler silo 904 can be interchangeably referred to as the inner wall. The traveler silo is sized and shaped to receive a head portion of the traveler 112, such that a portion of the head of the traveler contacts the inner surface of the traveler silo 904. The traveler silo 904 extends along a length of the rotatable platform. As the rotatable platform is rotated, the interaction between the traveler silo 904 inner surface and the causes the traveler 112 to rotate, and due to the threaded or other connection between the flaps and the traveler 112, the traveler 112 moves linearly upward, pushing the plunger 110 upward. The head of the traveler 112 moves upward as well, traveling along the length of the traveler silo 904.

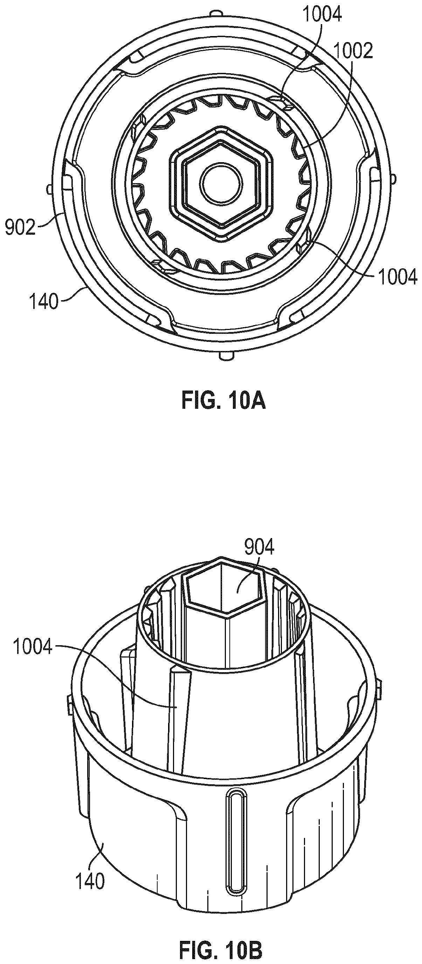

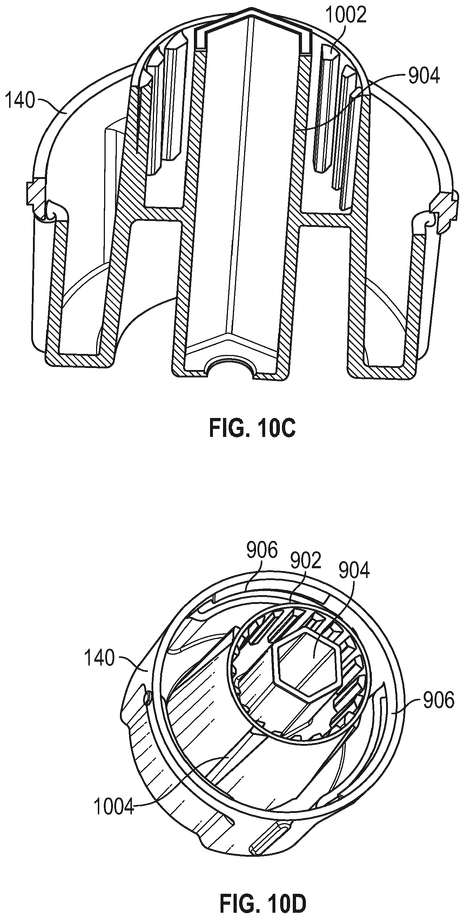

FIGs. 10A, 10B, and 10D each depict various angled views of rotatable platform 140. Moreover, FIG. 10C depicts a cross sectional, side view of the rotatable platform 140. As shown in FIGs. 10A-10D, in some embodiments the rotatable platform 140 further comprises inner side ticks 1002 and outer side ticks 1004. Inner side ticks 1002 are configured to interact with primary ticker tabs 202 to produce sounds and haptic feedback when the rotatable platform 140 is turned. In some embodiments, the rotatable platform 140 comprises outer side ticks 1004, the outer side ticks are configured to interact with secondary ticker tabs 308 to produce sounds and haptic feedback.

As shown in FIGs. 10B and 10C, the outer side ticks 1004 arise from the outside of the middle wall 804, while on the opposite side of middle wall 804, inner side ticks 1002 are present on the outer surface of the middle wall 804. In one embodiment, shown in for example FIGS. 10A and 10D, the middle wall includes 20 inner side ticks 1002 such that they are spaced apart equally every 18 degrees around the inner surface of the middle wall. As explained above, the primary ticker tabs 202 can protrude perpendicularly downward from the bottom wall 210 of the housing complex 108, such that they can rest in the slots between the inner side ticks 1002. As the rotatable platform 140 is rotated, the primary ticker tabs 202 bend as they pass over the inner side ticks 1002 until they snap into the adjacent slot, causing an audible "click." The inner side ticks 1002 may be spaced in such a way that at every 18 degrees of displacement of the rotatable platform 140, a first sound is created by the simultaneous fall of the primary ticker tabs 202 as they pass a corresponding inner side tick 1002. In one embodiment shown in, for example, FIGS. 10A and 10D, the middle wall includes 4 outer side ticks 1004 such that they are spaced apart equally every 90 degrees around the outer surface of the middle wall. At every 90 degrees, a second sound is created by the movement of the secondary ticker tabs 308 over the outer side ticks 1004, which can be concurrent with the first sound. For example, when the inner side ticks 1002 are spaced apart 18 degrees and the outer side ticks 1004 are spaced apart 90 degrees, the second sound will be created on every fifth of the first sounds and can be concurrent with that first sound.

The inner side ticks 1002 and/or primary ticker tabs 202 can be of a certain shape to facilitate a desired interaction between the rotatable platform 140 and the housing complex 108, such as an anti-back rotation mechanism. In some embodiments, the inner side ticks 1002, and/or the outer side ticks 1004 are shaped to prevent backwards movement of the primary ticker tabs 202 and/or secondary ticker tabs 308. For example, the inner side ticks 1002 and/or the outer side ticks 1004 may comprise any number of surfaces that would allow for movement of the primary ticker tabs 202 and/or secondary ticker tabs 308 across the ticks unidirectionally, but would be raised sufficiently to prevent or hinder backwards or reverse rotational movement of the rotatable platform 140. For example, as shown in FIGs. 10A and 11A, the inner side ticks 1002 can have a sloped front edge on one side which allows the primary ticker tabs 202 to slide up and over the sloped edge, but also have a sharp back edge, which can prevent backward rotation of the driver complex 170 when attached to the housing complex 108.

In some embodiments, such as particularly shown in FIGs. 10D and 11A, the rotatable platform 140 can include a plurality of ridges 906 around the inside top edge of the outer wall 802. The ridges 906 can correspond to the area over each of acoustic voids 902. In other embodiments, the rotatable platform 140 includes a singular ridge that extends around the circumference of the outer wall 802. As explained below, the ridges 906 provide an attachment mechanism for connecting the rotatable platform 140 with the housing complex 108, particularly the end lip 150.

In some embodiments, the acoustic voids 902 prolong the sound at every 90 degrees, to make it easier to understand dispensed dosage. The presence of acoustic voids 902 allows for a 2-10 microsecond delay, or more preferably, a 2-3 microsecond delay between the first sound and the second sound at every 90 degrees. The net effect of the microsecond delay is a crisper, more noticeably distinct sound to ensure the correct dispensing dosage.

As can be appreciated in FIGs. 9C, 10B, 10C, and 10D, a traveler silo 904 is formed from the structure of the inner wall 806. In some embodiments, the traveler silo 904 is configured to guide and house the traveler 112 when placed within the rotatable platform 140.

In some embodiments, there are 20 inner side ticks 1002 present on the rotatable platform 140. In some embodiments, there are 1, 2, 3, 4, 5, 6, 7, 8, 9, 10, 15, 25, 30, 40, 50, 60, 70, 80, 90, or any value in between the aforementioned inner side ticks 1002 present on the rotatable platform 140. In some embodiments, there are 1, 2, 3, 4, 5, 6, 7, 8, 9, 10, 15, 25, 30, 40, 50, 60, 70, 80, 90, or any value in between the aforementioned outer side ticks 1004 present on the rotatable platform 140. In some embodiments, inner side ticks 1002 can comprise any surface feature sufficient to generate a noise in conjunction with primary ticker tabs 202 when middle wall 804 is rotated.

FIG. 11A depicts an angled view of rotatable platform 140 along with traveler 112. Similarly, FIG. 11B depicts a cross section view of FIG. 11A. In some embodiments, the traveler 112 is fixed in place relative to the rotatable platform 140 using a traveler locking head 1102 (see also FIG. 14A), such that rotation of the rotatable platform corresponds to rotation of the traveler 112. As shown in FIGs. 11A and 14A, the traveler 112 can include a shaft 1104 extending from the locking head 1102 and a tip 1106 at the top end of the traveler 112. In some embodiments, the shaft 1104 is threaded. In some embodiments, the traveler locking head 1102 is a geometric shape, including any polygonal shape, including a triangle, a square, a rectangle, a pentagon, a hexagon, a heptagon, an octagon, a nonagon, or a decagon. In some embodiments, the traveler locking head 1102 shape corresponds to the shape of the traveler silo 904 within the inner wall 806. In this way, the traveler silo 904 can restrict free rotational movement of the traveler independent of the rotatable platform 140, but force rotational movement of the traveler 112 when the rotatable platform 140 is rotated. The relative fit of the traveler 112 can be loose enough to permit free up and down translational movement of the traveler 112 within the traveler silo 904. The fit of the traveler locking head 1102 relative to the traveler silo 904 of the inner wall 806 can be optimized to frictionally fit but not translationally constrain the traveler 112 in the traveler silo 904.

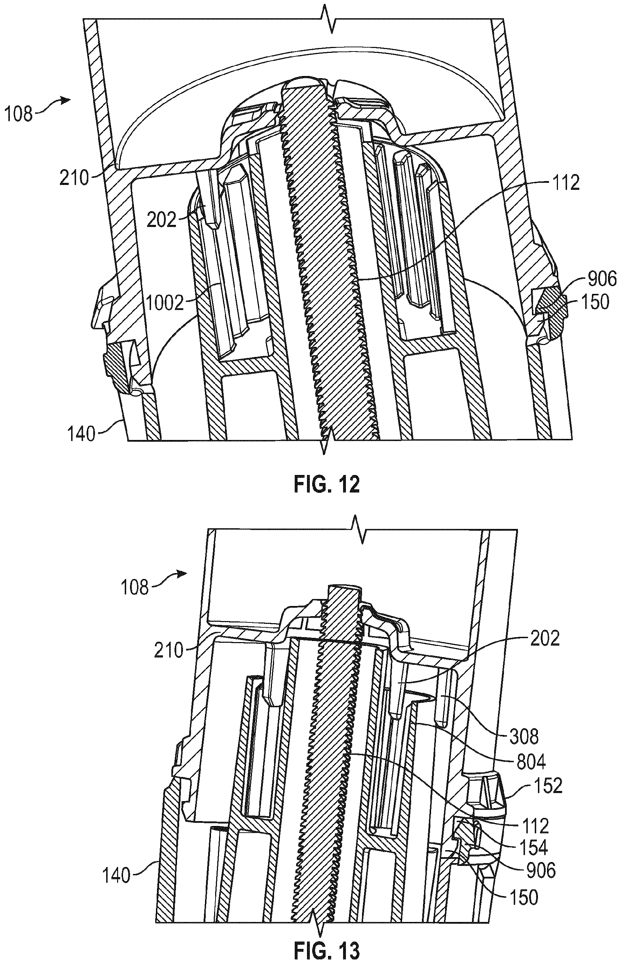

FIGs. 13-15, each depict cross section views of an assembled apparatus comprising the housing complex 108, traveler 112, and rotatable platform 140. Therefore, when the consumer applies force and rotates rotatable platform 140, as shown in FIGs. 12-14, primary ticker tabs 202 interact with inner side ticks 1002 to generate a first noise at predetermined locations or predetermined angular displacement values. Moreover, secondary ticker tabs 308 interact with outer side ticks 1004, to produce a second sound, depending on the spacing and position of the outer side ticks 1004 on the middle wall 804. As a result, distinct haptic and auditory feedback is provided to a consumer, allowing for greater ease of distribution and measuring of the composition to be dispensed. In some embodiments, upon an 18° displacement, primary ticker tabs 202 can clear inner side ticks 1002 and produce the first sound, and upon a 90° displacement, secondary ticker tabs 308 can clear outer side ticks 1004 and produce the second sound.

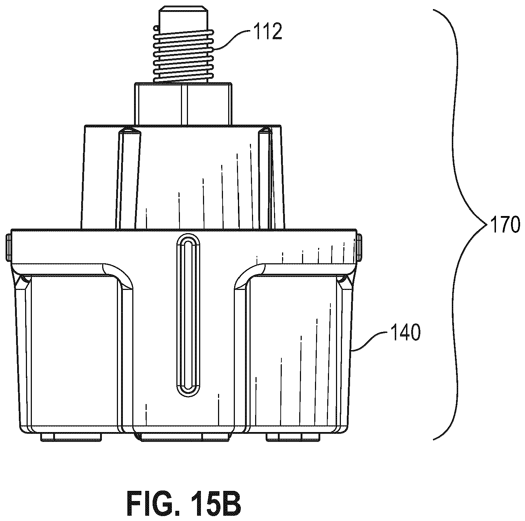

As explained above with respect to FIG. 1, the metered dose dispenser 100 can include a variety of components. In terms of the functionality of the components, it is helpful to consider some of the component parts together. FIGS. 15A and 15C show a set of components that can be considered a mixing complex 160. FIG. 15B shows another set of components that can be considered a driver complex 170. The metered dose dispenser 100 is the combination of the mixing complex 160 and the driver complex 170, with, in certain circumstances, the further addition of other components as explained herein. An important aspect of the disclosure is that the housing complex 108 can be used to first mix a medicament, then with other components, the same housing complex 108 can be used to dispense the medicament. In this manner, there is no need to transfer materials from a container used for mixing to a different container used for dispensing the medicament. Thus, the housing complex 108 itself is important functionally for mixing the medicament because the housing complex 108 can receive the medicament within the housing complex 108 in the upper chamber 142. The plunger 110 is placed within the upper chamber 142 before the medicament is added. The housing complex 108 together with at least the plunger 110 can be considered a mixing complex 160. Other components of the apparatus can also be considered part of the mixing complex 160. For example, in some embodiments such as shown in FIG. 15A, the volume indicator 114 can also be placed, along with the plunger 110 within the upper chamber 142 of the housing complex 108 before the medicament is added, thus the plunger 110 and the volume indicator 114 can be part of the mixing complex 160. In some embodiments, such as shown in FIG. 15C (and discussed below), a mixing lid 1702 can be placed on the housing complex 108 and can be considered part of the mixing complex 160. With the mixing complex 160 assembled such as shown in FIGs. 15A and 15C, the portion of the upper chamber 142 above the plunger 110 is a reservoir 162 for holding a substance such as a medicament for mixing and/or dispensing. Another advantage of the mixing complex 160, is the ability to easily prime (i.e. pre-set the stored volume of medicament) the unit, once the mixing has been completed. Laboratory personnel or other end users could simply push on the bottom of the volume indicator 114 with a thin rod of a predetermined length, in order to prime the mixing complex 160 to a desired volumetric amount and before coupling the driver complex 170 onto the mixing complex 160. Additionally, the traveler itself may also be used for priming the mixing complex 160 until the mixed composition of the upper chamber 142 starts to come out from the upper end of the housing 108, through the outlet of the dispensing lid 102. At that point, the rotatable platform 140 can be coupled to the mixing complex 160.

FIG. 15B shows a driver complex 170, which is the combination of at least the rotatable platform 140 and the traveler 112. As explained above, the traveler 112 rests at the bottom of the traveler silo 904 formed by the inner wall 806 of the rotatable platform 140. To connect the driver complex 170 to the mixing complex 160, the rotatable platform 140 (with the traveler 112 inside) can be pressed upward against the lower end 148 of the housing complex 108. In doing so, as shown for example in FIGS. 12, 13, and 14A, the ridges 906 on the inner surface of the outer wall 802 of the rotatable platform 140 can be flexed and pushed over the end lip 150 on the housing complex 108 to snap the driver complex 170 in place onto the housing complex 108. Thus, in some embodiments, the driver complex 170 is fixedly connected to the housing complex 108 and not removable, but allowing for rotation of the driver complex 170 relative to the housing complex 108. As shown in FIG. 15C (and also FIG. 13), the housing complex 108 can also include a raised ring 152 above the end lip 150, which may be graduated with demarcation line markings 408; the housing complex 108 may therefore have a lower trough 154 between the end lip 150 and the raised ring 152. In some embodiments, the lower trough 154 is sized such that when the rotatable platform is secured to the housing complex 108 over the end lip 150, the top rim of the rotatable platform 140 is pressed against and abuts the bottom of the raised ring 152, thereby creating a secure connection and fit between the rotatable platform 140 and the housing complex 108.

Referring to FIG. 13, when the driver complex 170 is connected to the housing complex 108, the middle wall 804 is positioned between the primary ticker tabs 202 (positioned on the inside of middle wall 804) and the secondary ticker tabs 308 (positioned on the outside of the middle wall 804). Thus, it will be understood that the middle wall 804 of the rotatable platform 140 has a radius from the central longitudinal axis of the housing 108 that is greater than the radius of the primary ticker tabs 202 and less than the radius of the secondary ticker tabs 308.

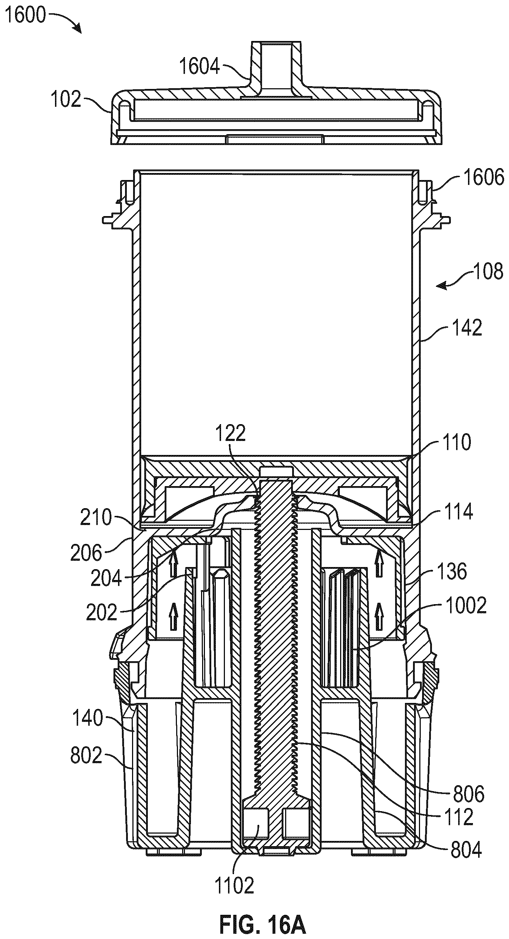

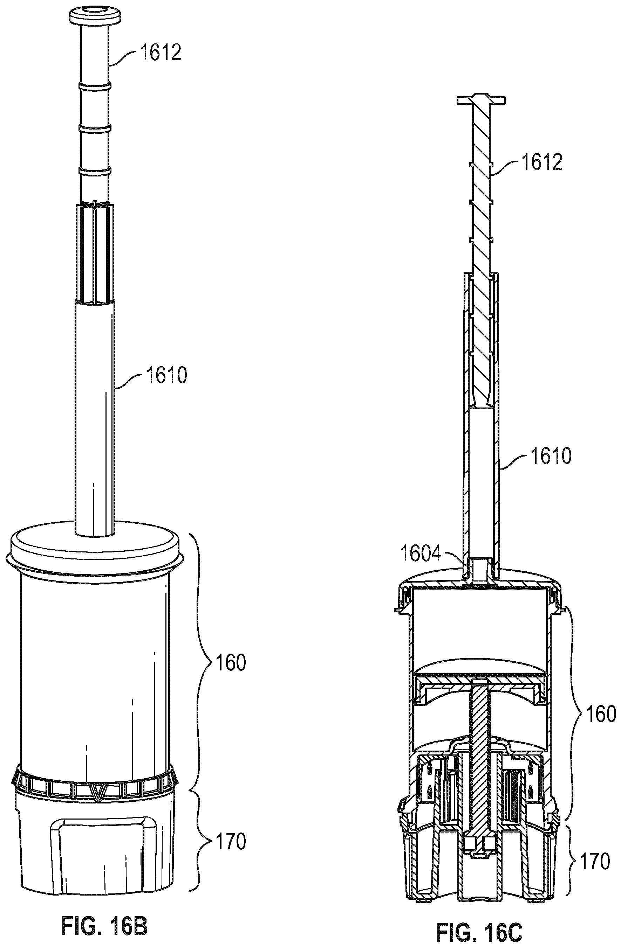

Referring to FIGs. 14A and 14B (see also FIG. 16), the components of the dispenser are sized such that when the driver complex 170 is attached to the housing complex 108 and when the traveler 112 is resting at the bottom of the traveler silo 904 (inner wall 806), the tip 1106 of the traveler 112 may extend through the bottom wall 210 to initially contact the bottom of the plunger 110 (or bottom of the volume indicator 114 if utilized). A portion of the shaft may be initially positioned in the flaps 204. A top rim of the inner wall 806 may extend into the aperture 208 in the bottom wall 210 and may reach the same level as the bottom wall 210 without extending into the upper chamber 142.

Referring to FIGs. 14A and 14B, the rotatable platform 140 can include, in some embodiments, a traveler access aperture 908 in the bottom of the inner wall 806 that passes through to the traveler silo 904. This traveler access aperture 908 allows access to the traveler 112, particularly the head 1102, in the traveler silo 904. In some embodiments it may be advantageous to advance the plunger 110 upward without rotating the rotatable platform 140 to rotate the traveler 112 through the bottom wall 210. This may be accomplished by, for example, pushing a rod (not shown) or other tool through the traveler access aperture 908 to push upward on the head 1102 of the traveler 112, thereby forcing the threaded shaft 1104 to push past the flaps 204 and grip the threads of the threaded shaft 1104 at a lower portion. When the user is done pushing the traveler 112 upwards, the flaps 204 will fall back into place on the threaded shaft 1104. In this manner, by pushing the plunger 110 linearly upward (non-rotationally), extraneous air may be quickly expelled or purged from the reservoir in the upper chamber 142 above the plunger 110. This process of forcing the plunger 110 linearly upward (non-rotationally) also allows for priming the flow of the substance in the reservoir.

FIG. 16A depicts a profile view of a combined apparatus comprising mixing complex 160 attached to driver complex 170 comprising the rotatable platform 140 and traveler 112. As can be appreciated, a medicament or other flowable composition may be stored in a reservoir 162 in the upper chamber 142, and driver complex 170 may be attached to mixing complex 160. With the attachment of a suitable dispensing lid 102, the entire apparatus can be used to dispense medicaments or flowable compositions, which is driven by the movement of the traveler 112, plunger 110, and volume indicator 114 upwards to upper end 146. Medicament then exits the apparatus via dispensing outlet 1604, which may be dispensed directly onto the skin, or other area where medicament is to be dispensed. In some embodiments, the dispensing outlet 1604 is a single outlet, whereas in other embodiments, the dispensing lid 102 includes a plurality of apertures for dispensing the medicament. In some embodiments, and as described in FIGs. 16B and 16C, the medicament may further be dispensed to secondary containers, for later application of volumes of medicament. With each rotation of the rotatable platform 140 to move the inner side ticks 1002 around the primary ticker tabs 202 into the next slot (e.g., an 18 degree rotation), the device will "click," and with that rotational movement the plunger 110 will be pushed up by the traveler 112 by a pre-determined distance to dispense a pre-determined amount of the medicament.

FIGs. 16B and 16C depicts profile views of a combined apparatus comprising mixing complex 160 attached to driver complex 170, and further attached to barrel 1610. In some embodiments, the combined assembly comprising mixing complex 160 and driver complex 170 can dispense medicaments into secondary containers (i.e. separate medical applicators), including secondary volumetric containers, for later application. In some embodiments, as depicted in FIGs 16B and 16C, a syringe like container comprising barrel 1610 and plunger 1612 can be fitted to accept medicaments poured from the apparatus. As shown in FIG. 16C, dispensing outlet 1604 is configured to dispense medicaments and other compounds within upper chamber 142 to the interior of barrel 1610, which can later dispense the same medicament as desired by a medical practitioners or a patient. As can be appreciated, any suitable container may be attached to dispensing outlet 1604 for direct filling, or a suitable medicament may be dispensed from dispenser outlet 1604 into said container.

### Mixing - Internal Impeller and EMP (Electronic Mortar and Pestle) Mixers

In some embodiments, the contents within the housing complex 108 can be mixed using mixers relying on an Electronic Mortar and Pestle (EMP) system, or which otherwise uses an impeller to induce mixing. For example, electronic mortar and pestle mixers rely on a rotating rod and internal impeller to mix, homogenize, or emulsify certain compositions. With the addition of certain components to the housing complex 108, it becomes possible to utilize and mix compositions using certain specialized mixers, or introduce certain mixing attachments.

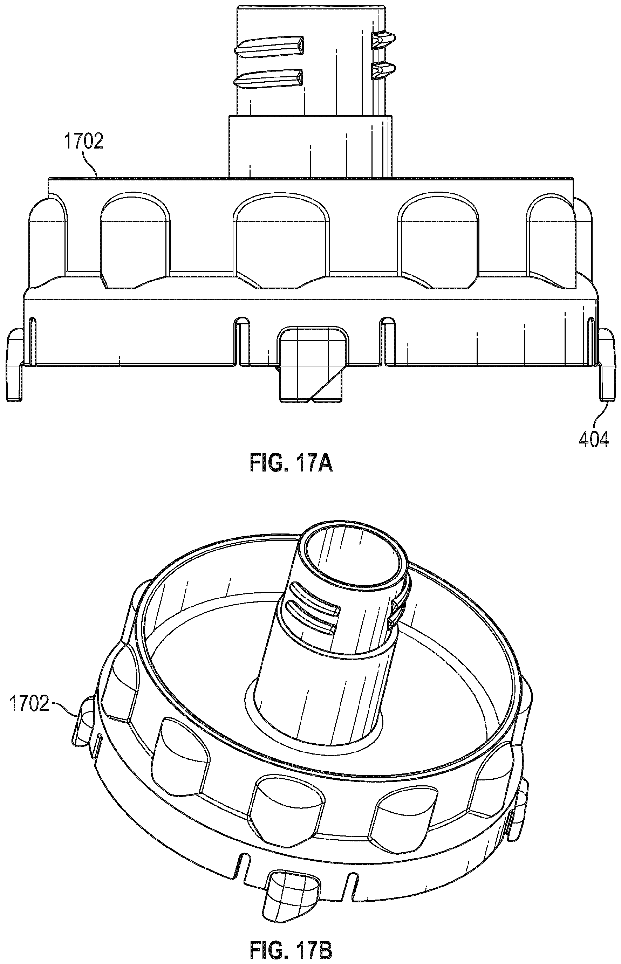

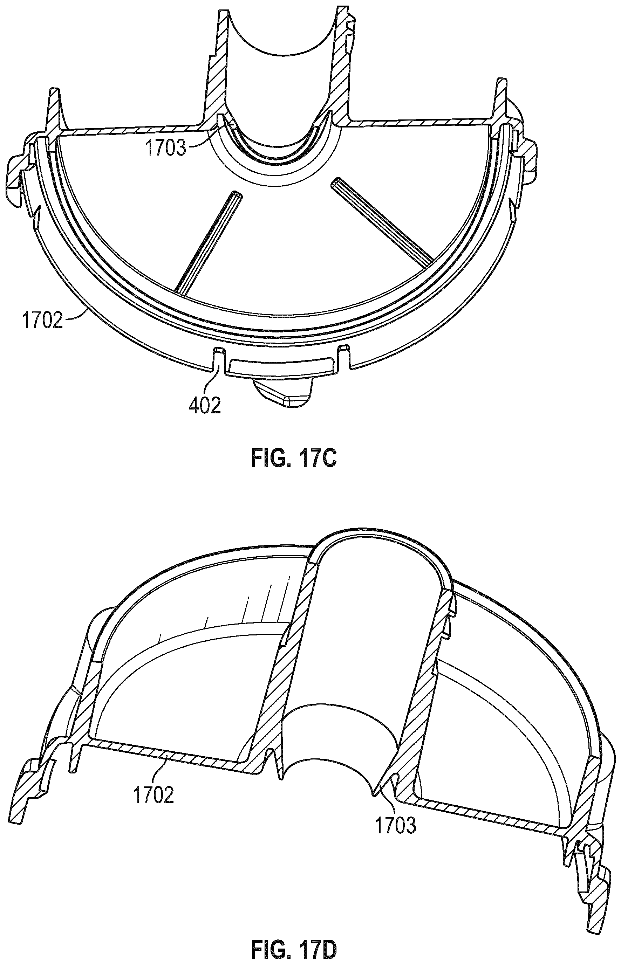

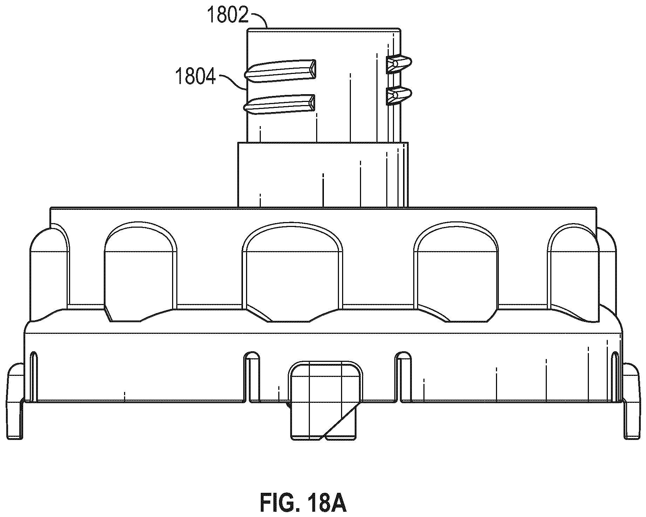

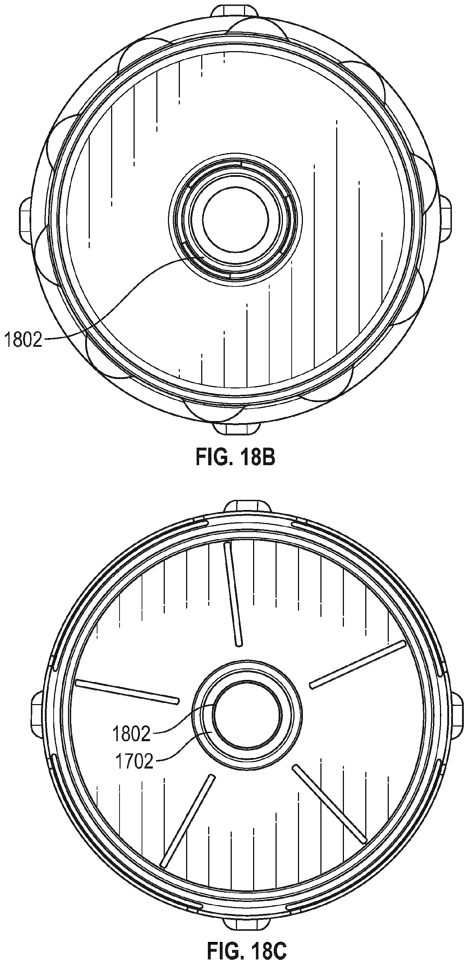

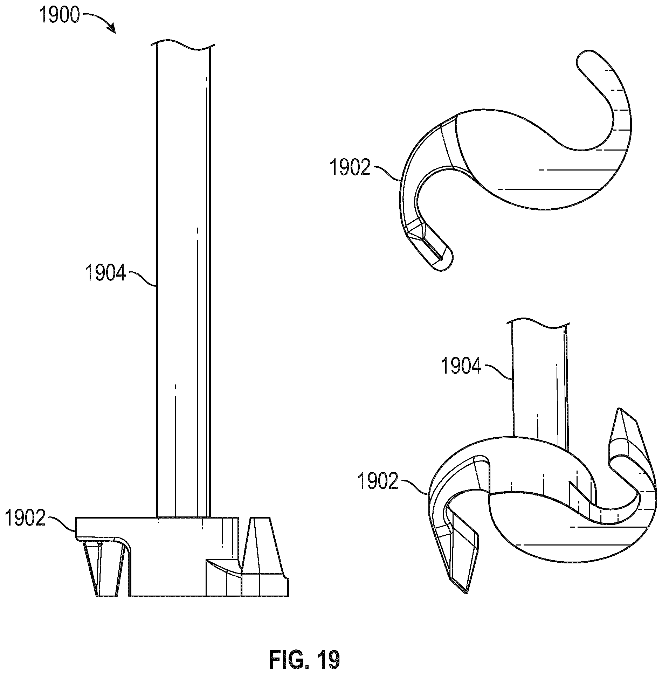

FIGs. 17A-D and 18A-C depict a mixing lid 1702 with varying angular and cross-sectional views. FIGs. 17A and B depict one embodiment of the mixing lid 1702, from which a lift notch system as described in FIG. 4 is shown, the mixing lid 1702 comprising vertical lift tab 404 and lift notches 402. FIG. 18B depicts a top down view of mixing lid 1702, while FIG. 18C depicts a bottom view of the same. In some embodiments, the mixing lid 1702 further comprises an impeller aperture 1802. FIG. 18A depicts a profile view of the mixing lid 1702, from which mixing lid threads 1804 are visible. In some embodiments, the mixing lid threads 1804 are configured to allow the lid to attach to a mixer. In some embodiments, the mixing lid 1702, and more specifically the impeller aperture 1802 and impeller seal 1703 , are configured to accept an impeller shaft 1904 as depicted in various views in FIG. 19. The impeller 1900 is comprised of the impeller shaft 1904 and an impeller tip 1902. In some embodiments, the impeller shaft 1904 is connected to an EMP mixer. In some embodiments, the impeller 1900 is configured to rotate in order to mix, homogenize, or emulsify a compound. In some embodiments, the impeller 1900 is configured to move up and down, to mix, homogenize, or emulsify a compound.

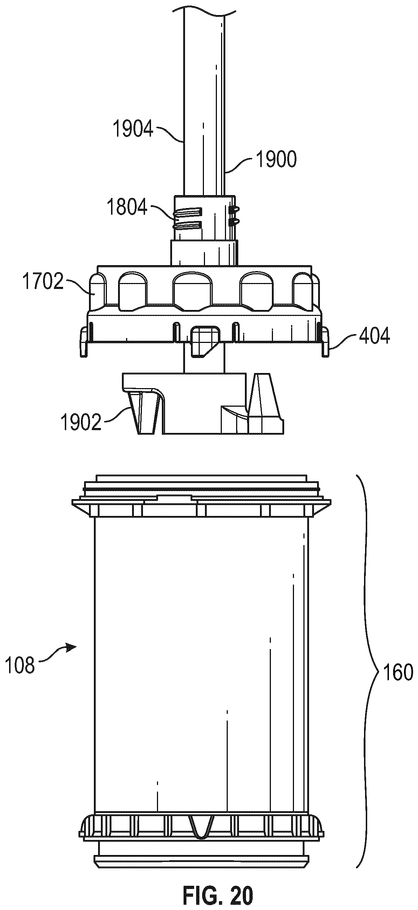

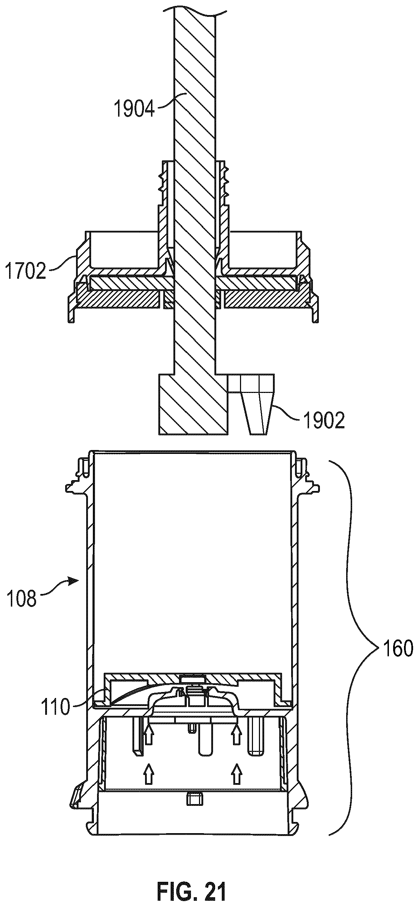

FIGs. 20 depicts an apparatus for mixing comprising the mixing complex 160 (including the housing complex 108 and at least the plunger 110), the mixing lid 1702, and the impeller 1900. FIG. 21 depicts a cross-sectional view of the same. In operation, an impeller 1900 slides vertically through the impeller aperture 1802, oscillating up and down while rotating axially to thoroughly mix a flowable composition and other pharmaceutical ingredients. The vertical movement of the impeller is bounded by the bottom wall of the upper chamber 142 of the housing complex 108 and by the mixing lid 1702. The mixing lid 1702 and the impeller seal 1703 are configured to create a secure attachment with the housing complex 108, such that no or minimal leakage of any compound or medicament within housing complex 108 is released while mixing. One end of impeller shaft 1904 is coupled to impeller tip 1902, and the opposite end is configured to be attached to a mixer, including EMP mixers. In some embodiments, the mixing lid 1702 can slide, snap, or screw onto the housing complex 108. In some embodiments, the impeller can traverse up and down relative to the height of the housing complex 108. FIG. 21 similarly depicts a cross section of a mixing assembly for use with EMP mixers.

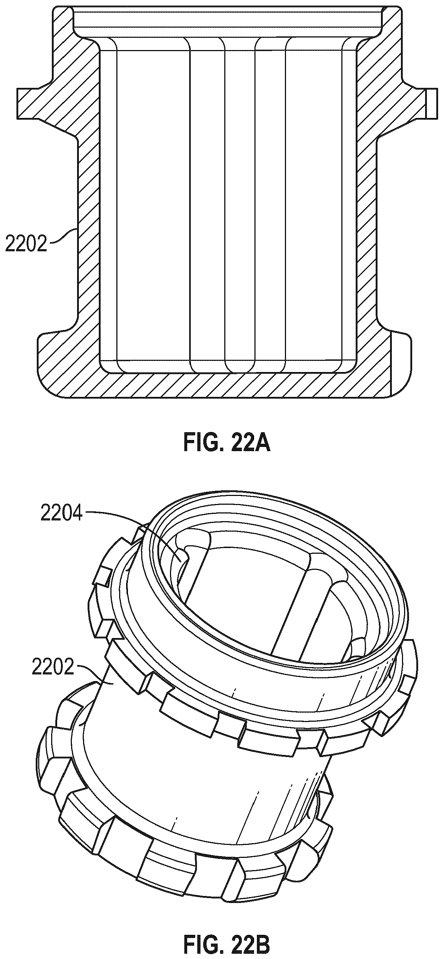

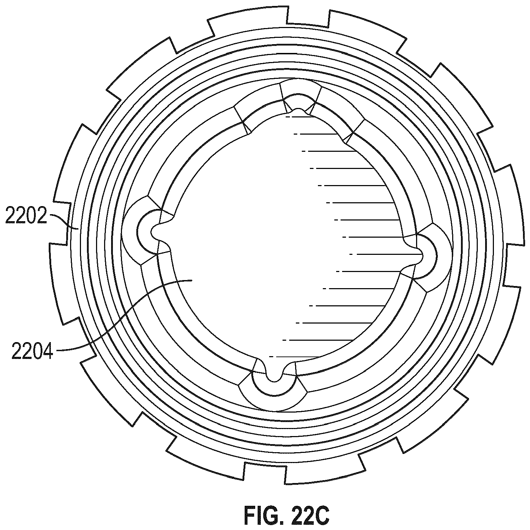

FIGs. 22A-C depicts various views of an adapter 2202. In some embodiments, an adapter 2202 may be utilized to secure the attachment of the housing complex 108 so that open receptacles or spaces within a mixer may be used. In some embodiments, the adapter 2202 may be utilized with an asymmetric mixer. In some embodiments, the adapter 2202 comprises a cylindrical inner cavity 2204, which is shaped to hold the housing complex 108 in place. In some embodiments, the adapter 2202 is configured to fit in any receptacle or slot on a mixer, to allow the housing complex 108 to be mixed. In some embodiments, the adapter 2022 includes multiple parts. In some embodiments, the adapter 2022 encloses the housing complex between one or more parts of the adapter 2022.

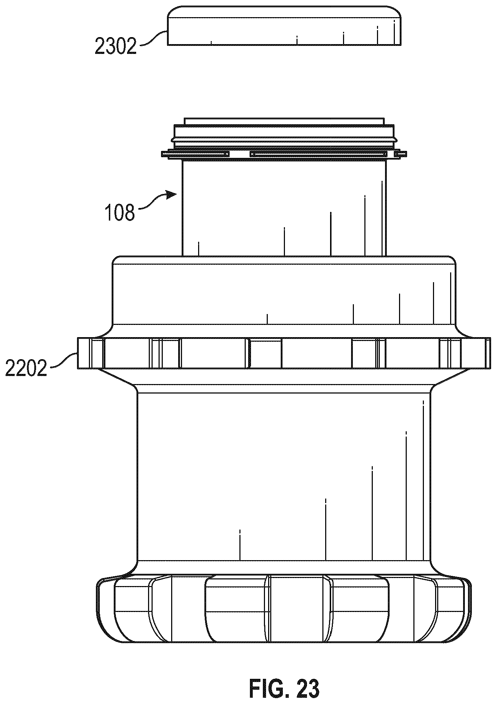

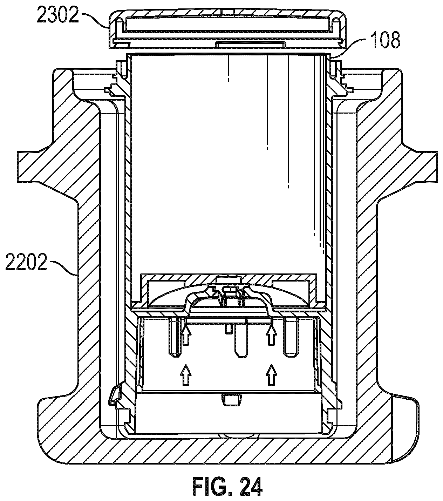

FIG. 23 depicts an assembly comprising adapter 2202, housing complex 108, and flat lid 2302. FIG. 24 depicts a cross section view of the assembly comprising adapter 2202, housing complex 108, and flat lid 2302. As can be appreciated, the housing complex 108 is securely fitted within adapter 2202, and more specifically within the cylindrical inner cavity 2204. A notable advantage of the assembly is the ability to use the housing complex 108 with a wide variety of mixer appliances, with rotors and bucket designs that may require specialized housings or adapters.

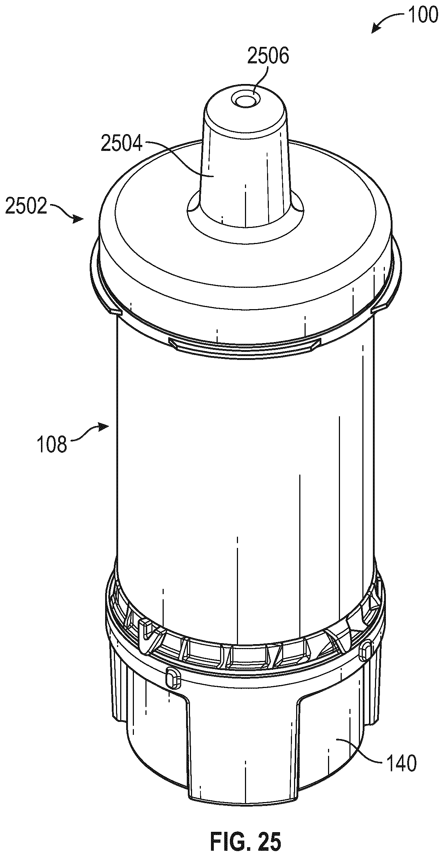

FIG. 25 an isometric view of a metered dose dispenser 100. The metered dose dispenser 100 may be similar or identical in many respects to the metered dose dispensers described above. The metered dose dispenser includes a dispensing lid 2502, a housing complex 108, and a rotatable platform 140. The dispensing lid 2502 may be similar or identical in many respects to the dispensing lid 102 discussed above. The dispensing lid 2502 includes a nipple 2504 that extends from a base of the dispensing lid 2502. The nipple 2504 includes a nipple aperture 2506. The nipple 2504 and nipple aperture 2506 are configured such that a fluid, solid, and/or medicament stored within the housing complex 108 is pushed from the housing complex 2506 through the nipple and out the nipple aperture 2506. The nipple 2504 may be beneficial for applying a medication to a hard to reach spot such as to the ear or ear canal.

The housing complex 108 may be similar in many respects to the housing complexes describe above. In some embodiments, the housing complex 108 may be configured to hold up to 20 ml of a fluid, solid, and/or medication.

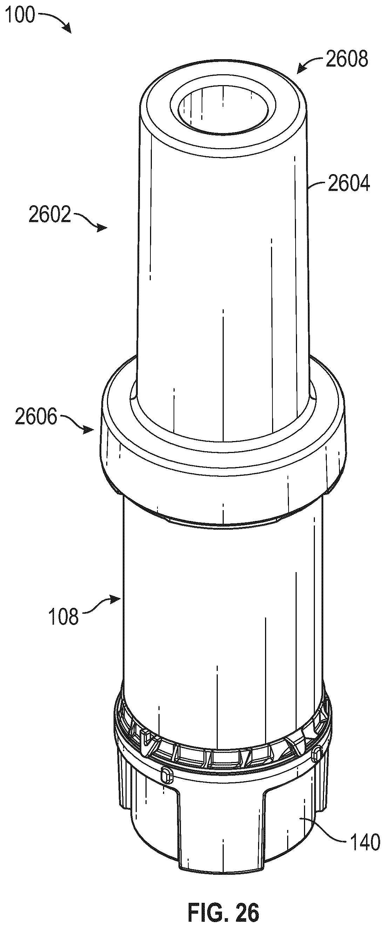

FIG. 26 depicts an isometric view of a metered dose dispenser 100 with a cap 2602. The cap includes a base 2606 and a top portion 2608. The cap 2062 is configured to be placed over the dispensing lid 2502. In some embodiments, the cap 2602 is configured to be secured onto the metered dose dispenser 100. In some embodiments, the cap 2602 may be secured onto the metered dose dispenser 100 via threads. In some embodiments the cap 2602 is configured to latch onto the metered dose dispenser 100. The cap 2602 includes a label surface 2604. The label surface 2604 may extend from the base 2606 of the cap 2602. The cap 2602 is cylindrical and larger than known caps in order to accommodate a prescription label. The label surface 2604 is configured such that a prescription label may be attached and placed on the label surface 2064. In some embodiments, where a small dispenser is used, the surface of the housing complex 108 may not be sufficiently large for a prescription label to be attached. The cap 2602 beneficially allows a prescription label to be attached to the label surface 2604.

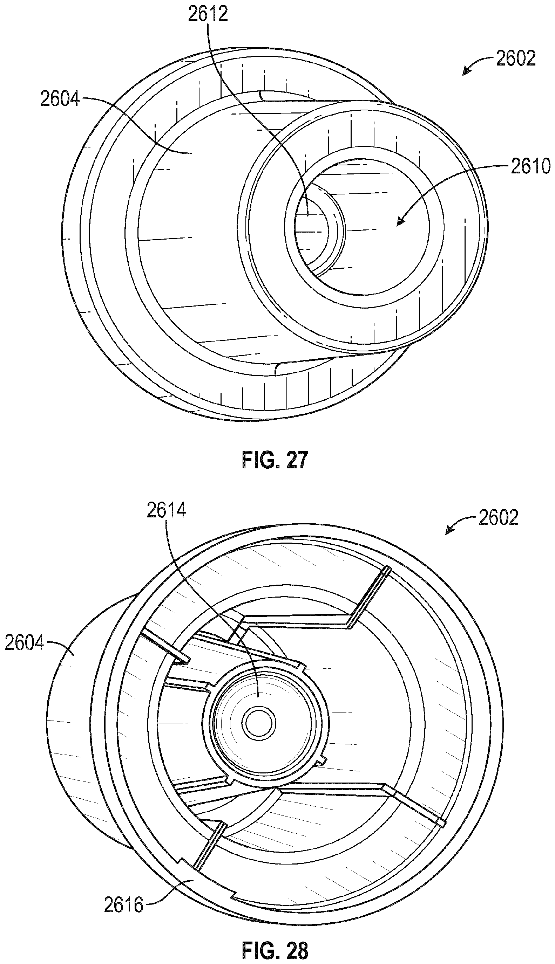

FIG. 27 depicts a top isometric view of a cap 2602. As seen in FIG. 27, the cap may include a cap aperture 2610. The cap aperture 2610 is circular in shape and begins at a top portion of the cap 2602 terminating at an aperture surface 2612. In some embodiments, the aperture surface is located at a midpoint between a top portion of the cap 2602 and a base of the cap 2602.

FIG. 28 depicts a bottom isometric view of a cap 2602. As seen in FIG. 28, the interior of the cap 2062 may be substantially hollow. The cap 2602 includes a cover surface 2614. The cover surface 2614 may extend from the top portion 2608 of the cap 2602. The cover surface 2614 is configured to cover the nipple aperture 2506 of the nipple 2504. In some embodiments, the cover surface 2614 prevents any liquid or solid contained within the housing complex 108 from exiting the housing complex 108 while the cap 2602 is secured to the metered dose dispenser 100. As seen in FIG. 28, the cap 2602 includes a latch 2616. The latch 2616 is configured to engage with a surface of the metered dose dispenser 100 and secure the cap 2602 to the metered dose dispenser 100. In some embodiments, the latch 2616 is configured to engage with a raised surface of the housing complex 108. In some embodiments, the latch 2616 is configured to engage with a surface of the dispensing lid 2502.

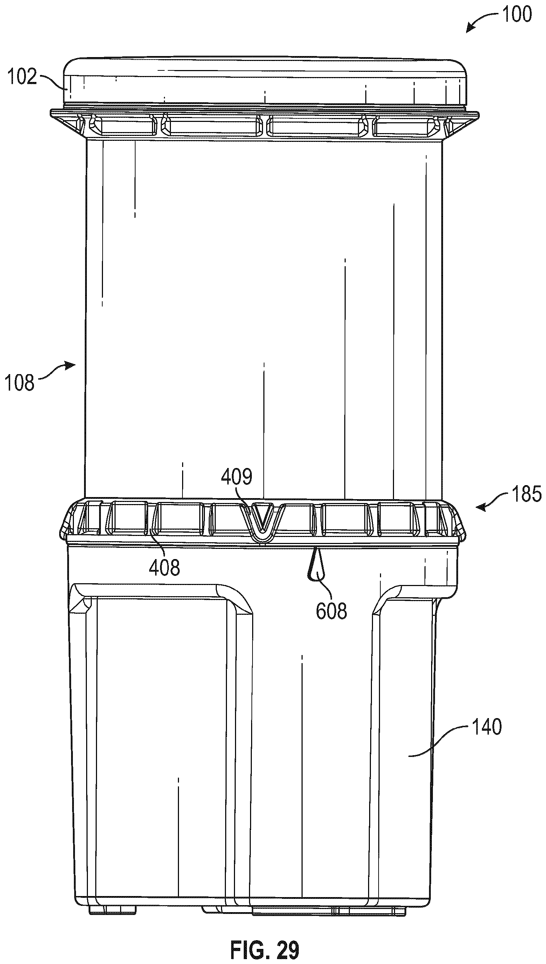

FIG. 29 depicts a front view of a metered dose dispenser 100. The metered dose dispenser 100 may be similar or identical in many respects to the metered dose dispensers described above. The metered dose dispenser 100 includes a dispensing lid 102, a housing complex 108, a raised ring 185, upper line marks 408, a rotatable platform 140, and lower line markings 608. The upper line markings 408 are disposed on the raised ring 185. As seen in FIG. 29, some or all of the upper line markings 408 may be the same shape and color. In some embodiments, each upper line marking 408 is a straight line. In some embodiments, some of the upper line markings 408 are different shapes or colors. In some embodiments, every fifth upper line mark 409 may be different in shape or color as the other upper line markings 408. In some embodiments, when the rotatable platform is rotated such that the lower line marking 608 lines up with a fifth upper line marking 409, both the primary and secondary ticker tabs interact with inner side ticks and outer side ticks respectively to produce a more accentuated sound. This arrangement using every fifth upper line mark 409 is exemplary only, and one of skill in the art would understand that other intervals could be advantageously used.

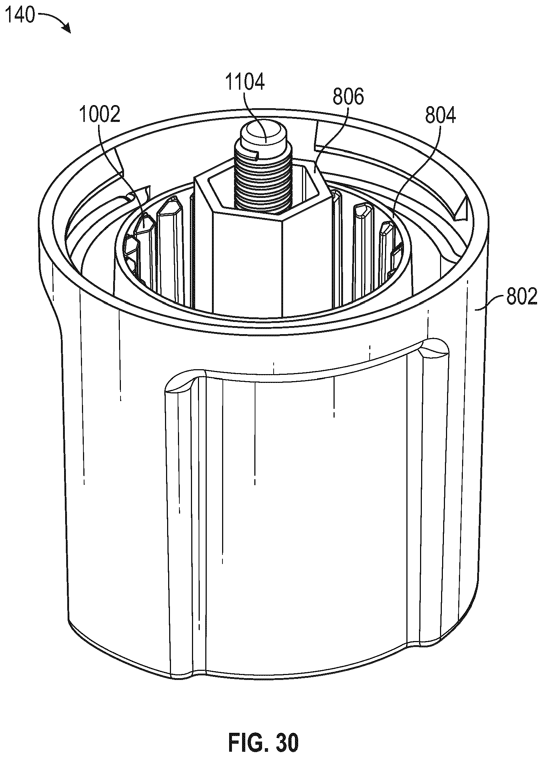

FIG. 30 shows an isometric view of a rotatable platform 140. The rotatable platform 140 may similar or identical respects to the rotatable platforms described above. As seen in FIG 30, the rotatable platform 140 may have an outer wall 802 that is taller than the outer walls described above. This may beneficially reduce the material needed for the outer housing 108. The features of the metered dose dispenser may be combined or substituted with any features described above in conjunction with different embodiments.

In various embodiments as described herein, example embodiments include at least the following aspects.

In some aspects, a dispensing apparatus includes: a housing including: an upper end; a lower end; a bottom wall positioned cross-sectionally between the upper end and the lower end the bottom wall having an aperture formed therein and a plurality of flaps surrounding the aperture and extending inward toward a center of the housing; and a plurality of primary ticks extending downward from the bottom wall of the housing; a plurality of secondary ticks extending downward from the bottom wall of the housing; a plunger positioned within the housing; a traveler in contact with the plunger, wherein the traveler extends through the aperture to move the plunger within the housing, and wherein the flaps are configured to engage the traveler; and a rotatable platform coupled to the lower end of the housing, the rotatable platform including an outer wall; an inner wall including: a plurality of inner side ticks disposed on an interior surface of the inner wall, the plurality of inner side ticks configured to interact with one or more of the plurality of primary ticks; and a plurality of outer side ticks disposed on an exterior surface of the inner wall, the plurality of inner side ticks configured to interact with the secondary ticks.

In some aspects, the rotatable platform further includes a traveler silo in contact with at least a portion of the traveler, such that rotation of the rotatable platform relative to the housing causes the traveler to rotate.

In some aspects, the traveler is configured to move upward in response to rotation of the rotatable platform through the aperture.

In some aspects, the traveler includes a head in contact with an inner surface of the traveler silo.

In some aspects, the traveler silo is configured to move upward in response to rotation of the rotatable platform through engagement between the head of the traveler and the inner surface of the traveler silo.

In some aspects, the inner wall of the rotatable platform is taller than the outer wall.

In some aspects, the traveler is longer than the length of the traveler silo.

In some aspects, a portion of the traveler silo extends into the aperture in the bottom wall of the housing.

In some aspects, the plurality of flaps includes four flaps positioned 90 degrees apart around the aperture.

In some aspects, a dispensing apparatus further includes a cap, the cap including a label surface of sufficient size for a prescription label to be affixed to the label surface.

In some aspects, a system for mixing and dispensing a medicament includes: a housing including: an upper chamber; a lower chamber; and a bottom wall dividing the upper chamber and the lower chamber, the bottom wall having an aperture formed therein and a plurality of flaps surrounding the aperture and extending inward toward a center of the housing; a plurality of primary ticks extending downward from the bottom wall of the housing; and a plurality of secondary ticks extending downward from the bottom wall of the housing, wherein a wall of the housing extends below the plurality of primary ticks and the plurality of secondary ticks, wherein the housing is configured to be inserted into a mixing apparatus, a bottom of the housing contacting a bottom of the mixing apparatus.

In some aspects, the system further includes: a driver complex including a rotatable platform and a traveler, wherein the driver complex is configured to be coupled to the mixing complex, wherein when the driver complex is coupled to the mixing complex, the plurality of flaps in the bottom wall are configured to engage the traveler, wherein rotating the rotatable platform causes the traveler to rotate upward through the aperture and move a plunger within the housing upward in the upper chamber of the housing and dispense the medicament through the at least one dispensing outlet.

In some aspects, the mixing complex further includes a volume indicator below the plunger in the upper chamber, the volume indicator including an indicator lip.

In some aspects, the system further includes a mixing lid to close the housing.

In some aspects, the mixing lid includes an impeller aperture and an impeller seal.

In some aspects, the mixing lid includes a plurality of vertical lift taps and a lift notch on each side of the lift tab.

In some aspects, the system further includes a dispensing lid coupled to the upper chamber, the dispensing lid including a dispensing outlet.

In some aspects, the mixing apparatus is a centrifugal mixer.

In some aspects, the techniques described herein relate to a method for dispensing a substance including: providing a substance within a housing of a dispensing apparatus; rotating a rotatable platform coupled to a lower end of the housing; moving in an upward direction, via the rotation of the rotatable platform, a traveler extending through an aperture of a bottom wall of the housing; engaging a plunger within the housing with the traveler; moving the plunger in an upward direction; and dispensing a substance through an aperture of a dispensing lid coupled to the upper end of the housing.

In some aspects, the techniques described herein relate to a method, the method further including: engaging a plurality of primary ticks extending from the bottom wall of the housing with a plurality of inner side ticks disposed on an interior surface of an inner wall of the rotatable platform; and creating a clicking sound caused by the engagement of the plurality of primary ticks with the plurality of inner side ticks as the rotatable platform rotates.

In some aspects, the techniques or devices described herein relate to a dispensing apparatus including: a housing including: an upper end; a lower end; a bottom wall positioned cross-sectionally between the upper end and the lower end, thereby forming an upper chamber and a lower chamber within the housing, the bottom wall having an aperture therethrough and a plurality of flaps extending inward toward a longitudinal axis through the center of the housing; and a plurality of primary ticks and secondary ticks extending downward from the bottom wall of the housing; a plunger positioned within the upper chamber of the housing; a traveler including a head, a threaded cylinder, and a tip, wherein the traveler is configured to extend through the bottom wall of the housing to move the plunger within the upper chamber of the housing, wherein the flaps in the bottom wall are configured to engage the threaded cylinder of the traveler as the traveler travels upwards through the bottom wall; a rotatable platform configured to be coupled to the lower end of the housing, the rotatable platform including concentric walls including: an outer wall that forms the exterior surface of the rotatable platform; a middle wall including a plurality of inner side ticks on an interior surface of the middle wall configured to interact with the primary ticker tabs extending from the bottom wall of the housing, and further including a plurality of outer side ticks on an exterior surface of the middle wall configured to interact with the secondary ticker tabs extending from the bottom wall of the housing; and an inner wall, wherein the inner wall forms a traveler silo configured to house at least a portion of the traveler.

In some aspects, the techniques or devices described herein relate to a dispensing apparatus, wherein axially rotating the rotatable platform relative to the housing is configured to axially rotate the traveler relative to the housing.

In some aspects, the techniques or devices described herein relate to a dispensing apparatus, wherein axially rotating the traveler moves the traveler upward within the traveler silo and rotates the traveler through the flaps in the bottom wall of the housing.

In some aspects, the techniques or devices described herein relate to a dispensing apparatus, wherein the traveler head includes a larger diameter than the diameter of the threaded cylinder and the tip, and wherein the traveler head includes a smaller diameter than the diameter of the traveler silo.

In some aspects, the techniques or devices described herein relate to a dispensing apparatus, wherein the traveler silo is sized to engage and rotate the traveler head and to permit translational upward movement of the traveler head within the traveler silo upon axially rotation of the rotatable platform.

In some aspects, the techniques or devices described herein relate to a dispensing apparatus, wherein the inner wall of the rotatable platform is taller than the middle wall and the middle wall is taller than the outer wall.

In some aspects, the techniques or devices described herein relate to a dispensing apparatus, wherein the inner wall is a hexagon shape, and wherein the head of the traveler is a hexagon shape.

In some aspects, the techniques or devices described herein relate to a dispensing apparatus, wherein the traveler is longer than the length of the traveler silo in the inner wall.

In some aspects, the techniques or devices described herein relate to a dispensing apparatus, wherein the inner wall of the rotatable platform includes a top rim, wherein when the apparatus is assembled the top rim is configured to extend into a plane of the aperture in the bottom wall of the housing.

In some aspects, the techniques or devices described herein relate to a dispensing apparatus, wherein the plurality of flaps includes four flaps, the flaps positioned 90 degrees apart around the aperture in the bottom wall.

In some aspects, the techniques or described herein relate to a dispensing apparatus comprising a cap, the cap configured to be secured to the housing of the dispensing apparatus, the cap including a label surface, wherein the label surface is of sufficient size for a prescription label to be affixed to the label surface.

In some aspects, the techniques or devices described herein relate to a system for mixing and dispensing a medicament including: a mixing complex configured to be received in a mixing apparatus for mixing the medicament, the mixing complex including a housing and a plunger, wherein the housing includes: an upper chamber; a lower chamber; and a bottom wall dividing the upper chamber and the lower chamber, wherein the bottom wall includes an aperture therethrough centered around a longitudinal axis of the housing, wherein the bottom wall includes a plurality of flaps extending toward the longitudinal axis, wherein the plunger includes an upper sealing lip and is positioned in the upper chamber of the housing and disposed above the bottom wall, wherein the sealing lip is configured to contact an interior surface of the housing, wherein the mixing complex is configured to receive one or more components of the medicament in the upper chamber above the plunger; a driver complex including a rotatable platform and a traveler, wherein the driver complex is configured to be coupled to the mixing complex, wherein the traveler includes a head and a threaded cylinder, wherein the rotatable platform includes a traveler silo and the traveler is configured to be at least partially disposed in the traveler silo, a dispensing lid including at least one dispensing outlet, wherein when the driver complex is coupled to the mixing complex, the flaps in the bottom wall are configured to engage the threaded cylinder of the traveler, wherein rotating the rotatable platform causes the traveler to rotate upward through the flaps and move the plunger upward in the upper chamber of the housing and dispense the medicament through the at least one dispensing outlet.

In some aspects, the techniques or devices described herein relate to a system, wherein the traveler silo is hexagon shaped and the head of the traveler is hexagon shaped.

In some aspects, the techniques or devices described herein relate to a system, wherein the rotatable platform includes concentric walls including: an outer wall that forms the exterior surface of the rotatable platform; and an inner wall that forms the traveler silo.

In some aspects, the techniques or devices described herein relate to a system, wherein the rotatable platform includes a middle wall as part of the concentric walls, the middle wall disposed between the outer wall and the inner wall.

In some aspects, the techniques or devices described herein relate to a system, wherein the inner wall is taller than the middle wall, and the middle wall is taller than the outer wall.

In some aspects, the techniques or devices described herein relate to a system, wherein the mixing complex further includes a volume indicator below the plunger in the upper chamber, the volume indicator including an indicator lip.

In some aspects, the techniques or devices described herein relate to a system, further including a mixing lid to close the housing complex when medicament is mixed in the housing complex.

In some aspects, the techniques or devices described herein relate to a system, wherein the mixing lid includes an impeller aperture and an impeller seal.

In some aspects, the techniques or devices described herein relate to a system, wherein the mixing lid includes a plurality of vertical lift taps and a lift notch on each side of the lift tab.

In some aspects, the techniques or devices described herein relate to a system, wherein the dispensing lid includes only one dispensing outlet and the dispensing outlet is configured to engage a separate medicinal applicator.

In some aspects, the techniques or devices described herein relate to a system, wherein around the exterior of the outer wall, upper line markings are arranged every 90 degrees, and wherein, around the exterior of the lower end of the housing, lower line markings are arranged every 18 degrees.

The illustrations of embodiments described herein are intended to provide a general understanding of the structure of various embodiments, and they are not intended to serve as a complete description of all the elements and features of components and systems that might make use of the structures described herein. Many other embodiments will be apparent to those of ordinary skill in the art upon reviewing the description provided herein. Other embodiments may be utilized and derived, such that structural and logical substitutions and changes may be made without departing from the scope of this disclosure. The figures herein are merely representational and may not be drawn to scale. Certain proportions thereof may be exaggerated, while others may be minimized. Accordingly, the specification and drawings are to be regarded in an illustrative rather than a restrictive sense.

The description herein may include terms, such as "up", "down", "upper", "lower", "first", "second", "primary", "secondary", "inner", "outer", etc. that are used for descriptive purposes only and are not to be construed as limiting. The elements, materials, geometries, dimensions, and sequence of operations may all be varied to suit particular applications. Parts of some embodiments may be included in, or substituted for, those of other embodiments. While the foregoing examples of dimensions and ranges are considered typical, the various embodiments are not limited to such dimensions or ranges.

The Abstract is provided to allow the reader to quickly ascertain the nature and gist of the technical disclosure. The Abstract is submitted with the understanding that it will not be used to interpret or limit the scope or meaning of the claims.

In the foregoing Detailed Description, various features are grouped together in a single embodiment for the purpose of streamlining the disclosure. This method of disclosure is not to be interpreted as reflecting an intention that the claimed embodiments have more features than are expressly recited in each claim. Thus, the following claims are hereby incorporated into the Detailed Description, with each claim standing on its own as a separate embodiment.

Although the disclosed subject matter has been described with reference to several example embodiments, it may be understood that the words that have been used are words of description and illustration, rather than words of limitation. Changes may be made within the purview of the appended claims, as presently stated and as amended, without departing from the scope and spirit of the disclosed subject matter in all its aspects. Although the disclosed subject matter has been described with reference to particular means, materials, and embodiments, the disclosed subject matter is not intended to be limited to the particulars disclosed; rather, the subject matter extends to all functionally equivalent structures, methods, and uses such as are within the scope of the appended claims.

## Claims

1. A dispensing apparatus comprising:
a housing comprising:
an upper end;
a lower end;
a bottom wall positioned cross-sectionally between the upper end and the lower end the bottom wall having an aperture formed therein and a plurality of flaps surrounding the aperture and extending inward toward a center of the housing; and
a plurality of primary ticks extending downward from the bottom wall of the housing;
a plurality of secondary ticks extending downward from the bottom wall of the housing;
a plunger positioned within the housing;
a traveler in contact with the plunger, wherein the traveler extends through the aperture to move the plunger within the housing, and wherein the flaps are configured to engage the traveler; and
a rotatable platform coupled to the lower end of the housing, the rotatable platform comprising
an outer wall;
an inner wall comprising:
a plurality of inner side ticks disposed on an interior surface of the inner wall, the plurality of inner side ticks configured to interact with one or more of the plurality of primary ticks; and
a plurality of outer side ticks disposed on an exterior surface of the inner wall, the plurality of inner side ticks configured to interact with the secondary ticks.

2. The dispensing apparatus of claim 1 wherein the rotatable platform further comprises a traveler silo in contact with at least a portion of the traveler, such that rotation of the rotatable platform relative to the housing causes the traveler to rotate.

3. The dispensing apparatus of claim 2, wherein the traveler is configured to move upward in response to rotation of the rotatable platform through the aperture.

4. The dispensing apparatus of claim 2, wherein the traveler comprises a head in contact with an inner surface of the traveler silo.

5. The dispensing apparatus of claim 4, wherein the traveler silo is configured to move upward in response to rotation of the rotatable platform through engagement between the head of the traveler and the inner surface of the traveler silo.

6. The dispensing apparatus of claim 1, wherein the inner wall of the rotatable platform is taller than the outer wall and/or wherein the traveler is longer than the length of the traveler silo and/or wherein a portion of the traveler silo extends into the aperture in the bottom wall of the housing and/or wherein the plurality of flaps comprises four flaps positioned 90 degrees apart around the aperture.

7. The dispensing apparatus of claim 1 further comprising a cap, the cap including a label surface of sufficient size for a prescription label to be affixed to the label surface.

8. A system for mixing and dispensing a medicament comprising:
a housing comprising:
an upper chamber;
a lower chamber; and
a bottom wall dividing the upper chamber and the lower chamber, the bottom wall having an aperture formed therein and a plurality of flaps surrounding the aperture and extending inward toward a center of the housing;
a plurality of primary ticks extending downward from the bottom wall of the housing; and
a plurality of secondary ticks extending downward from the bottom wall of the housing,
wherein a wall of the housing extends below the plurality of primary ticks and the plurality of secondary ticks, and
wherein the housing is configured to be inserted into a mixing apparatus.

9. The system of claim 8, further comprising:
a driver complex comprising a rotatable platform and a traveler, wherein the driver complex is configured to be coupled to the mixing complex,
wherein when the driver complex is coupled to the mixing complex, the plurality of flaps in the bottom wall are configured to engage the traveler, wherein rotating the rotatable platform causes the traveler to rotate upward through the aperture and move a plunger within the housing upward in the upper chamber of the housing and dispense the medicament through the at least one dispensing outlet.

10. The system of claim 8, wherein the mixing complex further comprises a volume indicator below the plunger in the upper chamber, the volume indicator comprising an indicator lip.

11. The system of claim 8, further comprising a mixing lid to close the housing.

12. The system of claim 11, wherein the mixing lid comprises an impeller aperture and an impeller seal and/or wherein the mixing lid comprises a plurality of vertical lift taps and a lift notch on each side of the lift tab.

13. The system of claim 8, wherein the system further comprises a dispensing lid coupled to the upper chamber, the dispensing lid comprising a dispensing outlet and/or wherein the mixing apparatus is a centrifugal mixer, an EMP mixer, or an impeller-based mixer.

14. A method for dispensing a substance comprising:
providing a substance within a housing of a dispensing apparatus;
rotating a rotatable platform coupled to a lower end of the housing;
moving in an upward direction, via the rotation of the rotatable platform, a traveler extending through an aperture of a bottom wall of the housing;
engaging a plunger within the housing with the traveler;
moving the plunger in an upward direction; and
dispensing a substance through an aperture of a dispensing lid coupled to the upper end of the housing.

15. The method of claim 14, the method further comprising:
engaging a plurality of primary ticks extending from the bottom wall of the housing with a plurality of inner side ticks disposed on an interior surface of an inner wall of the rotatable platform; and
creating a clicking sound caused by the engagement of the plurality of primary ticks with the plurality of inner side ticks as the rotatable platform rotates.
